# EUROPEAN PATENT SPECIFICATION

(11) **EP 4 547 662 B1**
(45) Date of publication and mention of the grant of the patent: **11.11.2026**
(21) Application number: 23736305.6
(22) Date of filing: 29.06.2023
(51) Int. Cl.: C07D 471/04, C07D 487/04, A61P 19/02, A61P 3/10, A61P 1/16, A61P 29/00, A61K 31/501, A61K 31/519

(54) **IMIDAZO[4,5-B]PYRIDINE AND PYRAZOLO[1,5-A]PYRIMIDINE DERIVATIVES AS SIK MODULATORS FOR THE TREATMENT OF RHEUMATOID ARTHRITIS**
IMIDAZO[4,5-B]PYRIDIN- UND PYRAZOLO[1,5-A]PYRIMIDIN-DERIVATE ALS SIK MODULATOREN ZUR BEHANDLUNG VON RHEUMATOIDER ARTHRITIS
DÉRIVÉS D'IMIDAZO[4,5-B]PYRIDINE ET PYRAZOLO[1,5-A]PYRIMIDINE EN TANT QUE MODULATEURS DE SIK POUR LE TRAITEMENT DE POLYARTHRITE RHUMATOÏDE

(30) Priority: 01.07.2022 EP 22182511
(43) Date of publication of application: 07.05.2025
(73) Proprietor: F. Hoffmann-La Roche AG, 4070 Basel (CH)
(72) Inventor: DÉCORET, Guillaume, 4070 Basel (CH); GROEBKE ZBINDEN, Katrin, 4070 Basel (CH); HAAP, Wolfgang, 4070 Basel (CH); KREIS, Lukas, 4070 Basel (CH); LUCAS CABRÉ, Xavier, 4070 Basel (CH); WACH, Jean-Yves, 4070 Basel (CH); WERMUTH, Roger, 4070 Basel (CH)
(74) Representative: Vitra, Hermeto
(86) International application number: PCT/EP2023/067758
(87) International publication number: WO 2024/003208

(56) References cited:
- WO-A1-2019/105886
- WO-A1-2020/239660
- WO-A1-2022/031928

## Description

The present invention relates to organic compounds useful for therapy and/or prophylaxis in a mammal, and in particular to compounds that modulate SIK activity.

The invention relates in particular to a compound of formula (I) 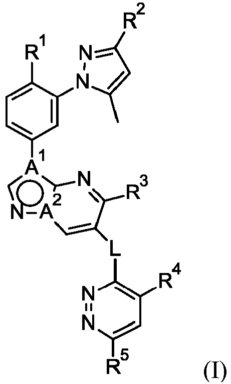 wherein
A¹ is -C- or -N-;
A² is -C- or -N-; with the proviso that if A¹ is -C-, then A² is -N-; and the proviso that if A¹ is - N-, then A² is -C-;
R¹ is cyano, hydroxyalkyl, alkylcarbonyl, haloalkoxy, hydroxy, alkoxy, halogen, haloalkyl, alkyl or cycloalkyl;
R² is cyano, hydroxyalkyl, haloalkoxy, hydroxy, alkoxy, halogen, haloalkyl or alkyl;
R³ is alkoxy, alkyl, haloalkyl, haloalkoxy, halogen or hydrogen;
R⁴ is hydrogen, alkyl, alkoxy, haloalkyl, alkylaminocarbonyl or dialkylaminocarbonyl;
R⁵ is hydrogen, alkyl, halogen, haloalkyl or alkoxy; and
L is -O- or -NH-;
or a pharmaceutically acceptable salt thereof.

Salt-inducible kinases (SIK) belong to a subfamily of AMP-activated protein kinases (AMPK) called AMPK-related kinases. There are three members, named SIK1, SIK2 and SIK3, that are broadly expressed. Their major biological role is to modify gene expression by controlling the phosphorylation and subcellular localization of two key classes of transcriptional regulatory factors: CRTCs (cAMP-regulated transcriptional coactivators) and class IIa HDACs (Histone deacetylases). Indeed, in basal state, both CRTCs and HDACs are phosphorylated by SIK kinases, and sequestered in the cytoplasm through interactions with their cytoplasmic chaperones 14-3-3. In response to extracellular cues that usually increase intracellular levels of cAMP, the SIK kinases' activity is inhibited, CRTCs and HDACs are no longer phosphorylated and are hence released from 14-3-3. They can therefore translocate into the nucleus and regulate gene expression (reviewed in Wein et al., Trends Endocrinol Metab. 2018 Oct;29(10):723-735).

In macrophages, the inhibition of SIK kinases leads to 1) CRTC3 shuttling to the nucleus and increasing the transcription of IL-10,; and 2) translocation of HDAC4/5 to the nucleus and subsequent deacetylation of NF-κB resulting in decreased transcription of pro-inflammatory cytokines (Clark et al., Proc Natl Acad Sci U S A. 2012 Oct 16;109(42):16986-91.).

Macrophages are critical to maintaining tissue homeostasis, mediating inflammation, and promoting the resolution of inflammation. To achieve this diversity of function, macrophages have the ability to "polarize" differently in response to environment cues. The two extreme phenotypes along their activation state continuum are the "M1" or "pro-inflammatory macrophages" and the "M2" or "pro-resolution macrophages".

Strikingly, the inhibition of intracellular SIK kinases overrides these extracellular macrophage polarization signals and pushes them toward a pro-resolution phenotype. This comes with an increase in IL-10 (by interfering with the SIK-CRTC3 pathway) and a concomitant decrease in TNF-α, IL-12 and IL-6 (by interfering with the SIK-HDAC4/5 and NF-κB pathway). The high levels of IL-10 and low levels of pro-inflammatory cytokines upon SIK inhibition will promote resolution of inflammation. The exploration of the SIK pathway has initially been described in macrophages (Clark et al., Proc Natl Acad Sci U S A. 2012 Oct 16;109(42):16986-91) and dendritic cells (Sundberg et al., Proc Natl Acad Sci U S A. 2014 Aug 26;111(34):12468-73) and the therapeutic potential of pan-SIK inhibitors has been confirmed in a mouse LPS (lipopolysaccharide) challenge model (Sundberg et al., ACS Chem Biol. 2016 Aug 19;11(8):2105-11) and in colitis models (Fu et al., Inflamm Bowel Dis. 2021 Oct 20;27(11):1821-1831). SIKs have since been shown to be important players in the functions of several immune cells, including mast cells (Darling et al., J Biol Chem. 2021 Jan-Jun;296:100428). Importantly, SIK1 is poorly expressed in macrophages and one embodiment of the invention are SIK2/3 inhibitors sparing SIK1, thus limiting potential SIK1-related toxicities.

SIK inhibitors have a high therapeutic potential in diseases that are 1) characterized by pro-inflammatory macrophage influx in the tissues and impaired tissue homeostasis and healing, or 2) where anti-TNF therapies are beneficial (partially or fully) or with insufficient levels of the IL10. Diseases with an inflammatory macrophage signature are e.g. rheumatoid arthritis, juvenile rheumatoid arthritis, NASH, primary sclerosing cholangitis, giant cell vasculitis and inflammatory bowel diseases ("IBD"), atherosclerosis, type 2 diabetes and glomerulonephritis.

Diseases with a proven link to IL-10 and TNF-α are IBD. Genetic alterations that reduce the function of IL-10 (such as SNPs in IL-10 or its receptor) are associated with an increased risk for IBD in humans. In addition, anti-TNF therapies are successful but only a subset of IBD patients are responsive and much of this limited responsiveness is lost over time. The described dual effect of SIK inhibitors (increased IL-10 and decreased TNF-α) make them particularly pertinent for the treatment of IBD.

All three SIK kinase isoforms are expressed broadly in human tissues with the highest expression observed in skin and adipose tissues for SIK1, adipose tissue for SIK2 and testis and brain for SIK3. Similarly to their role in macrophages, SIKs in these cells phosphorylate CRTCs and class II HDACs in response to extracellular signals, which subsequently change the expression of several cellular factors.

In addition to their physiological roles, reports have linked dysregulation of SIK expression to a few diseases. For example, SIK2 has been described as a risk locus for primary sclerosing cholangitis, a fibrotic disease regularly associated with IBD. In addition, SIK2 and SIK3 expression is higher in ovarian and prostate cancers and correlated with poor survival (Miranda et al., Cancer Cell. 2016 Aug 8;30(2):273-289; Bon et al., Mol Cancer Res. 2015 Apr;13(4):620-635).

As of today many diseases caused by dysregulation of the innate immune system lack efficient therapies and there is a high unmet medical need for new therapies. The present invention relates to a novel compounds that are highly active SIK inhibitors for the treatment of inflammatory, allergic and autoimmune diseases. In addition to inflammation, allergic and autoimmune diseases, SIK inhibitors can thus also be of potential relevance in cancer, metabolic diseases, bone density dysregulation diseases, pigmentation-related diseases or cosmetology, fibrotic diseases and depressive disorders.

WO 2022/031928 A1, WO 2020/239660 A1 and WO 2019/105886 A1 disclose fused N-heterocyclic compounds as SIK modulators for the treatment of e.g. rheumatoid arthritis.

In the present description the term "alkyl", alone or in combination, signifies a straight-chain or branched-chain alkyl group with 1 to 8 carbon atoms, particularly a straight or branched-chain alkyl group with 1 to 6 carbon atoms and more particularly a straight or branched-chain alkyl group with 1 to 4 carbon atoms. Examples of straight-chain and branched-chain C1-C8 alkyl groups include, but are not limited to, methyl, ethyl, propyl, isopropyl, butyl, isobutyl, tert.-butyl, the isomeric pentyls, the isomeric hexyls, the isomeric heptyls and the isomeric octyls, particularly methyl, ethyl, propyl, butyl and pentyl. Particular examples of alkyl are methyl, ethyl, propyl, isopropyl, butyl and isobutyl. Methyl, ethyl, propyl and butyl, like isobutyl, are further particular examples of "alkyl" in the compound of formula (I).

The term "cycloalkyl", alone or in combination, signifies a cycloalkyl ring with 3 to 8 carbon atoms and particularly a cycloalkyl ring with 3 to 6 carbon atoms. Examples of cycloalkyl include, but are not limited to, cyclopropyl, cyclobutyl, cyclopentyl, cyclohexyl, cycloheptyl and cyclooctyl. Particular examples of "cycloalkyl" are cyclopropyl and cyclobutyl.

The term "alkoxy" or "alkyloxy", alone or in combination, signifies a group of the formula alkyl-O- in which the term "alkyl" has the previously given significance, such as methoxy, ethoxy, n-propoxy, isopropoxy, n-butoxy, isobutoxy, sec-butoxy and tert.-butoxy. Particular examples of "alkoxy" are methoxy and ethoxy.

The term "oxy", alone or in combination, signifies the -O- group.

The terms "halogen" or "halo", alone or in combination, signifies fluorine, chlorine, bromine or iodine and particularly fluorine, chlorine or bromine, more particularly fluorine. The term "halo", in combination with another group, denotes the substitution of said group with at least one halogen, particularly substituted with one to five halogens, particularly one to four halogens, i.e. one, two, three or four halogens.

The term "haloalkyl", alone or in combination, denotes an alkyl group substituted with at least one halogen, particularly substituted with one to five halogens, particularly one to three halogens, more particularly two to three halogens. Particular "haloalkyl" are fluoromethyl, fluoroethyl, fluoropropyl, fluorobutyl, difluoromethyl, difluoroethyl, trifluoromethyl and trifluoroethyl.

The term "haloalkoxy", alone or in combination, denotes an alkoxy group substituted with at least one halogen, particularly substituted with one to five halogens, particularly one to three halogens. Particular "haloalkoxy" are fluoromethoxy, fluoroethoxy and fluoropropyloxy.

The terms "hydroxyl" and "hydroxy", alone or in combination, signify the -OH group.

The term "carbonyl", alone or in combination, signifies the -C(O)- group.

The term "amino", alone or in combination, signifies the primary amino group (-NH₂), the secondary amino group (-NH-), or the tertiary amino group (-N-).

The term "alkylamino" is an alkyl group linked to a -NH- group. The term "dialkylamino" denotes two alkyl groups linked to a -N- atom.

The term "alkylcarbonyl", is an alkyl group linked to a -C(O)- group. Particular "alkylcarbonyl" are methylcarbonyl (also known as acetyl) and ethylcarbonyl.

The term "sulfonyl", alone or in combination, signifies the -SO₂- group.

The term "pharmaceutically acceptable salts" denotes salts which are not biologically or otherwise undesirable. Pharmaceutically acceptable salts include both acid and base addition salts. The term "pharmaceutically acceptable acid addition salt" denotes those pharmaceutically acceptable salts formed with inorganic acids such as hydrochloric acid, hydrobromic acid, sulfuric acid, nitric acid, carbonic acid, phosphoric acid, and organic acids selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, p-toluenesulfonic acid, and salicyclic acid. The term "pharmaceutically acceptable base addition salt" denotes those pharmaceutically acceptable salts formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, N-ethylpiperidine, and polyamine resins.

The term "compound(s) of this invention" and "compound(s) of the present invention" refers to compounds of formula (I) and stereoisomers, tautomers, solvates, and salts (e.g., pharmaceutically acceptable salts) thereof.

Tautomeric forms, i.e. structural isomers which interconvert with the compound of formula (I), in particular in solution, may in some instances exist and are to be understood as being included in the invention.

If one of the starting materials or compounds of formula (I) contain one or more functional groups which are not stable or are reactive under the reaction conditions of one or more reaction steps, appropriate protecting groups (as described e.g. in "Protective Groups in Organic Chemistry" by T. W. Greene and P. G. M. Wuts, 3rd Ed., 1999, Wiley, New York) can be introduced before the critical step applying methods well known in the art. Such protecting groups can be removed at a later stage of the synthesis using standard methods described in the literature. Examples of protecting groups are tert-butoxycarbonyl (Boc), 9-fluorenylmethyl carbamate (Fmoc), 2-trimethylsilylethyl carbamate (Teoc), carbobenzyloxy (Cbz) and p-methoxybenzyloxycarbonyl (Moz).

The compound of formula (I) can contain several asymmetric centers and can be present in the form of optically pure enantiomers, mixtures of enantiomers such as, for example, racemates, mixtures of diastereoisomers, diastereoisomeric racemates or mixtures of diastereoisomeric racemates.

The term "asymmetric carbon atom" means a carbon atom with four different substituents. According to the Cahn-Ingold-Prelog Convention an asymmetric carbon atom can be of the "R" or "S" configuration.

Furthermore, the invention includes all optical isomers, i.e. diastereoisomers, diastereomeric mixtures, racemic mixtures, all their corresponding enantiomers and/or tautomers as well as their solvates, wherever applicable, of the compound of formula (I).

If desired, racemic mixtures of the compound of the invention may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography.

In the embodiments, where an optically pure enantiomer is provided, optically pure enantiomer means that the compound contains > 90 % of the desired isomer by weight, particularly > 95 % of the desired isomer by weight, or more particularly > 99 % of the desired isomer by weight, said weight percent based upon the total weight of the isomer of the compound. A chirally pure or chirally enriched compound may be prepared by chirally selective synthesis or by separation of enantiomers. The separation of enantiomers may be carried out on the final product or alternatively on a suitable intermediate.

Structures depicted herein are also meant to include compounds that differ only in the presence of one or more isotopically enriched atoms. Particular examples of radioisotopes are ²H, ³H, ¹³C, ¹⁴C and ¹⁸F. For example the structures wherein one or more hydrogen atoms are replaced by deuterium or tritium, or one or more carbon atoms are replaced by a ¹³C- or ¹⁴C-enriched carbon are within the scope of this invention.

The invention thus relates to:
A compound according to the invention wherein R¹ is cyano or hydroxyalkyl;
A compound according to the invention wherein R¹ is cyano or hydroxyethyl;
A compound according to the invention wherein R² is cyano, haloalkyl or haloalkoxy;
A compound according to the invention wherein R² is cyano, difluoromethyl or difluoromethoxy;
A compound according to the invention wherein R³ is hydrogen or alkoxy;
A compound according to the invention wherein R³ is hydrogen or methoxy;
A compound according to the invention wherein R⁴ is hydrogen or dialkylaminocarbonyl;
A compound according to the invention wherein R⁴ is hydrogen or dimethylaminocarbonyl;
A compound according to the invention wherein R⁵ is hydrogen or alkyl;
A compound according to the invention wherein R⁵ is hydrogen or methyl;
A compound according to the invention wherein A¹ is -C- and A² is -N-;
A compound according to the invention wherein A¹ is -N- and A² is -C-;
A compound according to the invention wherein L is -N-; and
A compound according to the invention wherein L is -O-;

The invention further relates to a compound of formula (I) selected from
1-[2-(1-hydroxyethyl)-5-[6-[(6-methylpyridazin-3-yl)amino]imidazo[4,5-b]pyridin-3-yl]phenyl]-5-methyl-pyrazole-3-carbonitrile;
1-[2-(1-hydroxyethyl)-5-[6-[(6-methylpyridazin-3-yl)amino]pyrazolo[1,5-a]pyrimidin-3-yl]phenyl]-5-methyl-pyrazole-3-carbonitrile;
1-[2-[3-(difluoromethyl)-5-methyl-pyrazol-1-yl]-4-[6-(pyridazin-3-ylamino)imidazo[4,5-b]pyridin-3-yl]phenyl]ethanol;
3-[[3-[4-cyano-3-[3-(difluoromethoxy)-5-methyl-pyrazol-1-yl]phenyl]-5-methoxy-imidazo[4,5-b]pyridin-6-yl]amino]-N,N,6-trimethyl-pyridazine-4-carboxamide;
1-[2-(1-hydroxyethyl)-5-[6-(6-methylpyridazin-3-yl)oxypyrazolo[1,5-a]pyrimidin-3-yl]phenyl]-5-methylpyrazole-3-carbonitrile;
3-[[3-[4-cyano-3-[3-(difluoromethoxy)-5-methyl-pyrazol-1-yl]phenyl]imidazo[4,5-b]pyridin-6-yl]amino]-N,N,6-trimethyl-pyridazine-4-carboxamide;
1-[3-fluoro-2-(1-hydroxyethyl)-5-[6-[(6-methylpyridazin-3-yl)amino]imidazo[4,5-b]pyridin-3-yl]phenyl]-5-methyl-pyrazole-3-carbonitrile; and
1-[2-[3-(difluoromethyl)-5-methyl-pyrazol-1-yl]-4-[6-[(6-methylpyridazin-3-yl)amino]pyrazolo[1,5-a]pyrimidin-3-yl]phenyl]ethanol;
or a pharmaceutically acceptable salt thereof.

The invention further relates in particular to a compound of formula (I) selected from
1-[2-(1-hydroxyethyl)-5-[6-[(6-methylpyridazin-3-yl)amino]imidazo[4,5-b]pyridin-3-yl]phenyl]-5-methyl-pyrazole-3-carbonitrile;
1-[2-(1-hydroxyethyl)-5-[6-[(6-methylpyridazin-3-yl)amino]pyrazolo[1,5-a]pyrimidin-3-yl]phenyl]-5-methyl-pyrazole-3-carbonitrile; and
1-[3-fluoro-2-(1-hydroxyethyl)-5-[6-[(6-methylpyridazin-3-yl)amino]imidazo[4,5-b]pyridin-3-yl]phenyl]-5-methyl-pyrazole-3-carbonitrile;
or a pharmaceutically acceptable salt thereof.

One embodiment of the invention relates to a compound according to the invention, wherein the compound is a compound of formula (II) 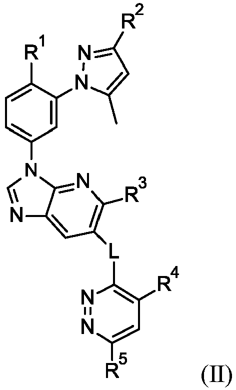 wherein L, R¹, R², R³, R⁴ and R⁵ are as described herein.

One embodiment of the invention relates to a compound according to the invention, wherein the compound is a compound of formula (III) 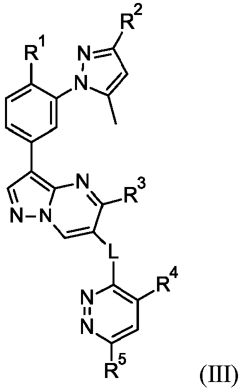 wherein L, R¹, R², R³, R⁴ and R⁵ are as described herein.

The synthesis of the compound of formula (I) can, for example, be accomplished according to the non-exhaustive procedures described below in general schemes 1-11 or according to methods known to those skilled in the art. In some instances, the sequence of the reaction steps can be modified and the individual steps of the different schemes can be combined in different ways as disclosed herein.

### Scheme 1

In scheme 1, the synthesis of a compound of formula (I-a) is described. In the scheme below L, A¹, A², R², R³, R⁴ and R⁵ are as defined above. The compound of formula (I-a) is a compound of formula (I) as described herein, wherein R¹ is hydroxyethyl.

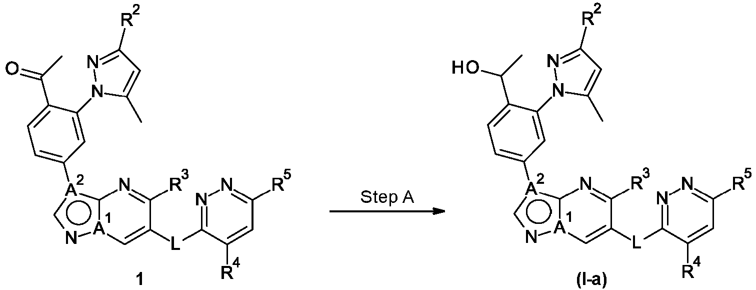

***Step A:*** Intermediate **1** can be converted to a compound of formula **(I-a)** with a reducing agent such as NaBH₄ in MeOH with a cosolvent such as THF, 1,4-dioxane, EtOH or dichloromethane at a temperature between -40 °C and RT.

### Scheme 2

In scheme 2, the synthesis of a compound of formula (I-a) is described. In the scheme below A¹ is -C- and A² is -N-; L, R², R³, R⁴ and R⁵ are as defined above. The compound of formula (I-b) is a compound of formula (I) as described herein, wherein R¹ is hydroxyethyl, A¹ is -C- and A² is -N-.

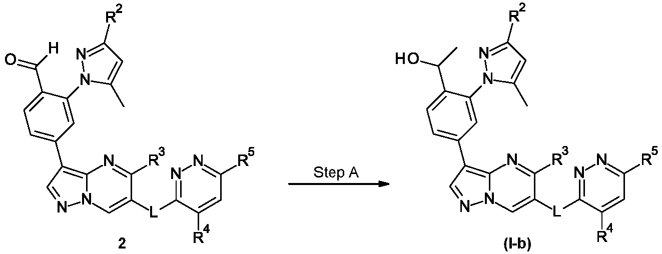

***Step A:*** Intermediate 2 can be converted to a compound of formula (I-b) by a Grignard reaction with methyl magnesium bromide in a solvent such as THF at a temperature of between about -70 °C and about 0 °C.

### Scheme 3

In scheme 3, the synthesis of a compound of formula (I-c) is described. In the scheme below A¹ is -N-, A² is -C- and L is -NH-; R¹, R², R³, R⁴ and R⁵ are as defined above. The compound of formula (I-c) is a compound of formula (I) as described herein, wherein A¹ is -N- and A² is -C-.

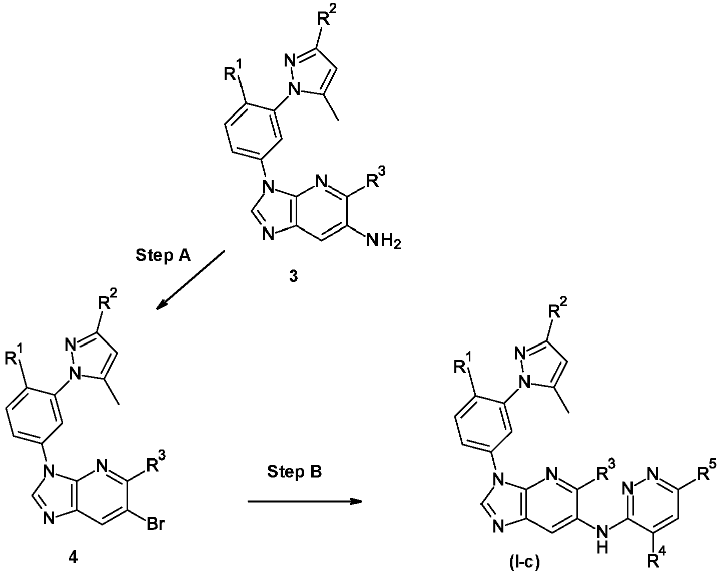

*Step A:* The intermediate **3** can be converted into the corresponding bromine intermediate **4** by a Sandmeyer reaction with a bromide source such as copper(I) dibromide, a nitrite source such as isoamyl nitrite in a solvent such as acetonitrile at temperatures between 60 °C and 80 °C.

*Step B:* A compound of formula **(I-c)** can be obtained via a Buchwald-Hartwig coupling of intermediate **4** with an aminopyridazine reactant using a suitable base such as for instance Cs₂CO₃, K₂CO₃ or K₃PO₄ and a suitable palladium catalyst such as for instance t-BuXPhos-Pd-G3 or [t-BuBrettPhos Pd(allyl)]OTf in a solvent such as 1,4-dioxane at temperatures between 80 °C and 100 °C.

### Scheme 4

In scheme 4, the synthesis of a compound of formula **(I-d)** is described. In the scheme below A¹ is -C-, A² is -N-, L is -NH- and R¹ is cyano, alkylcarbonyl or haloalkyl; R¹, R², R³, R⁴ and R⁵ are as defined above. The compound of formula (I-d) is a compound of formula (I) as described herein, wherein A¹ is -C- and A² is -N-.

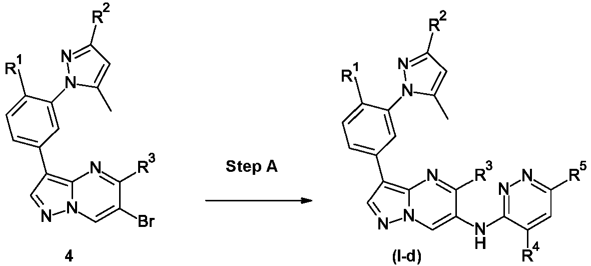

*Step A:* A compound of formula **(I-d)** can be obtained via a Buchwald-Hartwig coupling of intermediate **4** with an aminopyridazine reactant using a suitable base such as for instance Cs₂CO₃, K₂CO₃ or K₃PO₄, and a suitable palladium catalyst such as for instance t-BuXPhos-Pd-G3 or [t-BuBrettPhos Pd(allyl)]OTf in a solvent such as 1,4-dioxane at temperatures between 80 °C to 90 °C.

### Scheme 5

In the scheme below A¹ is -N-, A² is -C- and R¹ is cyano, alkylcarbonyl or haloalkyl; R², R³, R⁴ and R⁵ are as defined above.

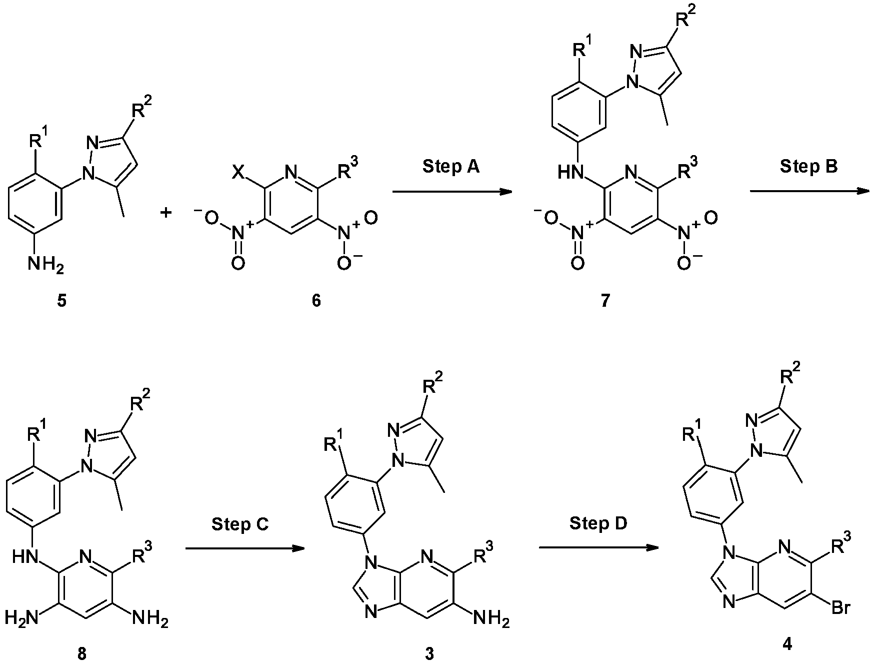

*Step A:* An intermediate **7** can be obtained by reacting an aniline **5** and a fluoro or chloropyridine **6** in a solvent such as methanol, THF, acetonitrile, DMF, NMP or DMSO at temperatures between around 0 °C and around 70 °C.

*Step B:* An aromatic amine intermediate **8** can be obtained by reduction of the corresponding nitro precursor **7** using a reduction such as Fe in a solvent such as EtOH or water in the presence of ammonium chloride or alternatively acetic acid at a temperature of 50 °C. Alternatively, the reduction can be achieved with Raney nickel or Pd/C as catalyst.

*Step C:* An intermediate **3** can be obtained from an aniline intermediate **8** by condensation with trimethoxymethane in the presence of TsOH in a solvent such as EtOH or MeOH at temperatures between around 80 °C and around 120 °C.

*Step D:* An aniline intermediate **3** can be converted into the corresponding bromine intermediate **4** by a Sandmeyer reaction with a bromide source such as copper(II) dibromide, a nitrite source such as isoamyl nitrite in a solvent such as acetonitrile at temperatures between around 60 °C and around 80 °C.

### Scheme 6

In the scheme below A¹ is -N-, A² is -C- and R¹ is cyano, alkylcarbonyl or haloalkyl; R², R³, R⁴ and R⁵ are as defined above.

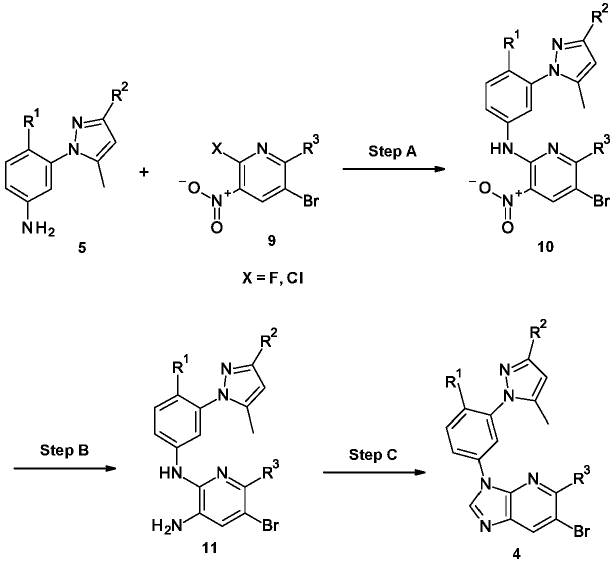

*Step A:* An intermediate **10** can be obtained by reacting an aniline **5** and a fluoro or chloropyridine **9** in a solvent such as MeOH, THF, acetonitrile, DMF or DMSO at temperatures between around 0 °C and around 70 °C. Deprotonation of the aniline might be required, for example using a base such as lithium bis(trimethylsilyl)amide in a suitable aprotic solvent.

*Step B:* An aniline intermediate **11** can be obtained by reduction of the corresponding nitro precursor **10** using a reduction such as Fe in a solvent such as EtOH or water in the presence of ammonium chloride at a temperature of around 50 °C.

*Step C:* An intermediate **4** can be obtained for aniline intermediate **11** by condensation with trimethoxymethane in the presence of TsOH in a solvent such as MeOH at temperatures between around 80 °C and around 120 °C.

### Scheme 7

In the scheme below A¹ is -N-, A² is -C- and R¹ is cyano, alkylcarbonyl or haloalkyl; R², R³, R⁴ and R⁵ are as defined above.

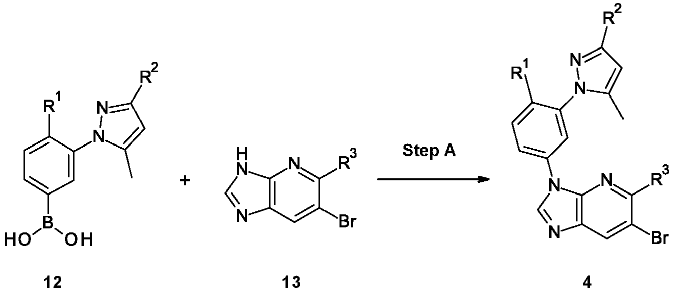

*Step A:* An intermediate 4 can be obtained by Chan-Lam coupling of a boronic acid intermediate 12 and an azabenzimidazole intermediate 13 in the presence of Cu(OAc)₂ in a solvent such as MeOH, EtOH, dichloromethane or acetonitrile under an oxygen atmosphere at temperatures between around 50 °C and around 100 °C.

### Scheme 8

In the scheme below A¹ is -C-, A² is -N- and R¹ is cyano, alkylcarbonyl or haloalkyl; R², R³, R⁴ and R⁵ are as defined above.

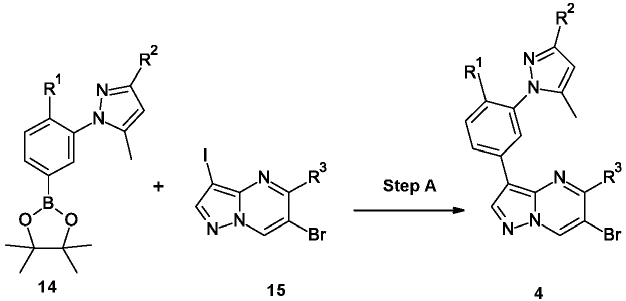

*Step A:* An intermediate **4** can be obtained by a Suzuki coupling between boronic ester **14** and a iodo precursor **15** in the presence of a catalyst such as Pd(dppf)Cl₂·CH₂Cl₂ complex and a base such as K₂CO₃ or Cs₂CO₃ in a solvent such as 1,4-dioxane, THF, DMF, DMA, NMP, toluene, H₂O or mixtures thereof at temperatures between around 50 °C and around 80 °C.

### Scheme 9

In the scheme below A¹ is -C-, A² is -N- and R¹ is cyano, alkylcarbonyl or haloalkyl; L, R², R³, R⁴ and R⁵ are as defined above. In the scheme below X is a halogen, preferentially bromo. The compound of formula (I-e) is a compound of formula (I) as described herein, wherein A¹ is - C-, A² is -N- and L is -NH-. The compound of formula (I-f) is a compound of formula (I) as described herein, wherein A¹ is -C-, A² is -N- and L is -O-.

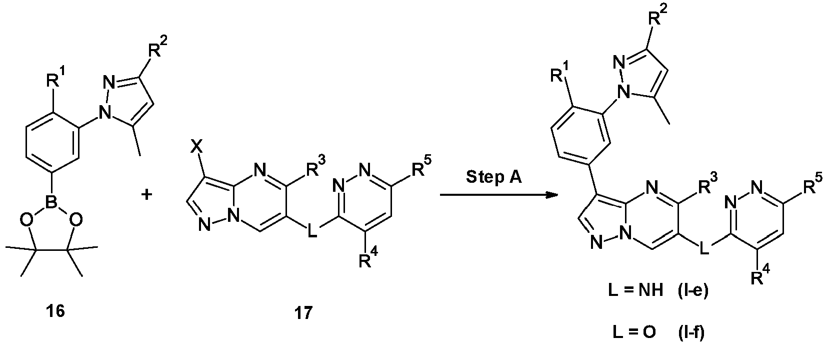

*Step A:* A compound of formula **(I-e)** or **(I-f)** can be obtained by Suzuki coupling of a boronic acid **16** and a halo precursor **17** in the presence of a catalyst such as Pd(dppf)Cl₂·CH₂Cl₂ complex and and a base such as K₂CO₃ or Cs₂CO₃ in a solvent such as 1,4-dioxane, THF, DMF, DMA, NMP, toluene, H₂O or mixtures thereof at temperatures between around 50 °C and around 80 °C.

### Scheme 10

In the scheme below L, R², R³, R⁴ and R⁵ are as defined above.

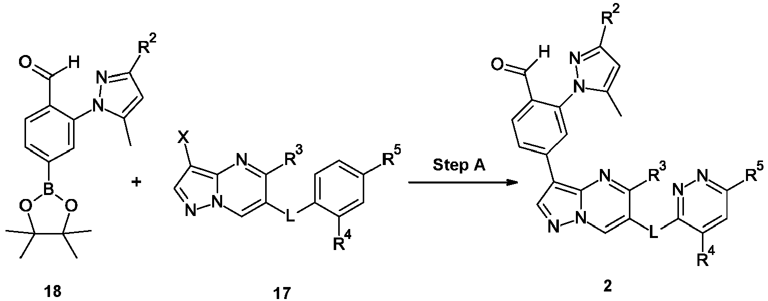

*Step A:* Intermediate **2** can be obtained by Suzuki coupling of a boronic ester **18** and a halo precursor **17** in the presence of a catalyst such as Pd(dppf)Cl₂·CH₂Cl₂ complex and and a base such as K₂CO₃ or Cs₂CO₃ in a solvent such as 1,4-dioxane, THF, DMF, DMA, NMP, toluene, H₂O or mixtures thereof at temperatures between around 50 °C and around 80 °C.

### Scheme 11

In the scheme below R¹ is cyano, alkylcarbonyl or haloalkyl; R² is as defined above.

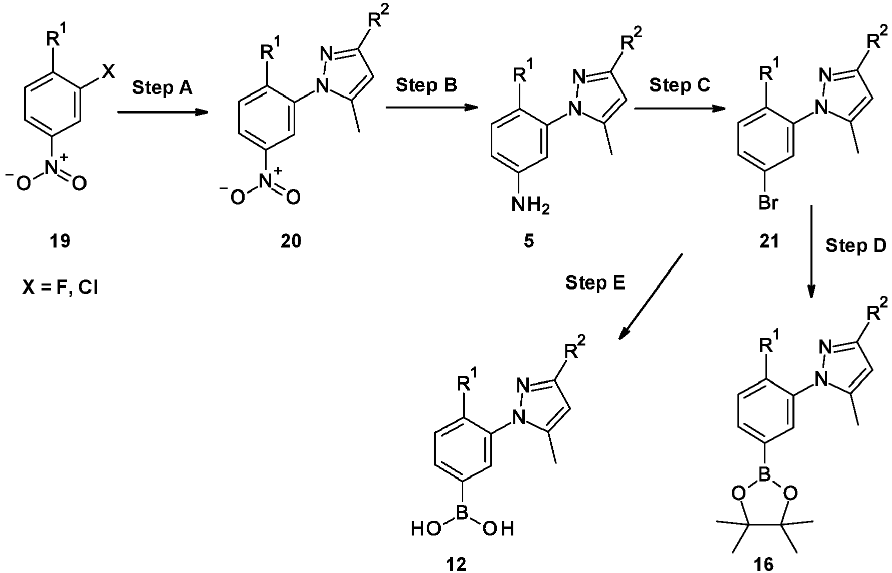

*Step A:* An intermediate **20** can be obtained by reacting the corresponding fluoro or chloro precursor **19** with a suitable pyrazole building block in the presence of a base such as DIPEA, NaHCO₃, K₂CO₃, Cs₂CO₃ or DBU in a solvent such as DMSO, DMF at temperatures between around 50 °C and around 100 °C.

*Step B:* An aniline intermediate **5** can be obtained by reduction of the corresponding nitro precursor **20** using a reduction such as Fe in a solvent such as EtOH or water in the presence of ammonium chloride.

*Step C:* An aniline intermediate **5** can be converted into the corresponding bromine intermediate **21** by a Sandmeyer reaction with a bromine source such as copper(II) dibromide, a nitrite source such as isoamyl nitrite in a solvent such as acetonitrile at temperatures between around 60 °C and around 80 °C.

*Step D:* A boronic ester intermediate **16** can be obtained by reaction a bromo intermediate **21** with bis(pinacolato)diboron in the presence of a catalyst such as Pd(dppf)Cl₂ and a base such as K₂CO₃ or KOAc in a solvent such as DMSO or 1,4-dioxane at a temperature of around 90 °C.

*Step E:* A boronic acid intermediate **12** can be obtained by reaction a bromo intermediate **21** with bis(pinacolato)diboron in the presence of a catalyst such as Pd(dppf)Cl₂ and a base such as K₂CO₃ or KOAc in a solvent such as DMSO, 1,4-dioxane at a temperature of around 90 °C.

### Scheme 12

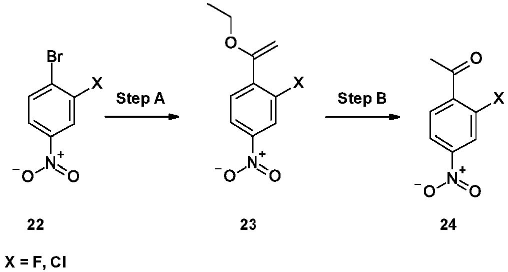

*Step A:* The alkoxyvinyl intermediate **23** can be obtained by a Stille coupling of precursor **22** with a tributyl(1-alkohoxvinyl)tin reagent in the presence of a palladium catalyst such as bis(triphenylphosphine)palladium(II) chloride in a solvent such as toluene, DMF or THF at temperatures ranging between around 80 °C and around 110 °C.

*Step B:* The acetyl intermediate **24** can be obtained by acidic hydrolysis of intermediate **23** using for example 1N aqueous HCl at RT in a suitable solvent such as for instance THF.

### Scheme 13

In the scheme below R² is as defined above.

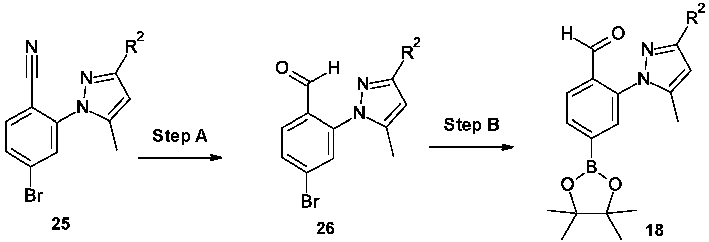

*Step A:* An intermediate **26** can be obtained by reacting the corresponding cyano precursor **25** with DIBAL-H in a solvent such as toluene, THF, dichloromethane, hexanes, n-heptane or a mixture thereof at temperatures between around -70 °C and around 0 °C.

*Step B:* A boronic ester intermediate **18** can be obtained by reaction a bromo intermediate 26 with bis(pinacolato)diboron in the presence of a catalyst such as Pd(dppf)Cl₂ and a base such as K₂CO₃ or KOAc in a solvent such as DMSO, 1,4-dioxane at a temperature of around 90 °C.

The invention thus also relates to a process for the preparation of a compound according to the invention, comprising one of the following steps:
(a) the reaction of a compound of formula (B1) 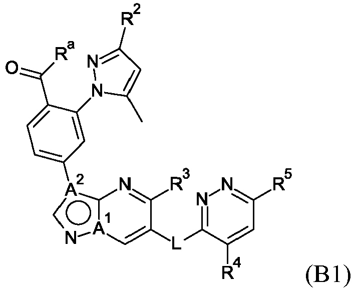 with a suitable reduction agent in presence of a suitable solvent,
(b) the reaction of a compound of formula (B2) 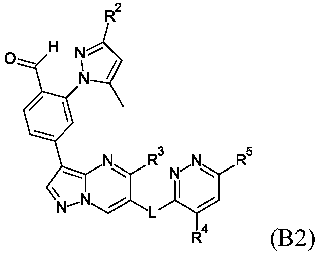 with a suitable Grignard reagent in presence of a suitable solvent,
(c) the reaction of a compound of formula (B3) 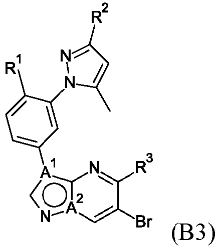
   with a compound of formula (B4) 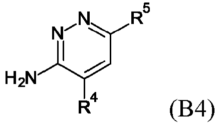
   in presence of a suitable base, a suitable catalyst and a suitable solvent, or
(d) the reaction of a compound of formula (B5) or (B5') 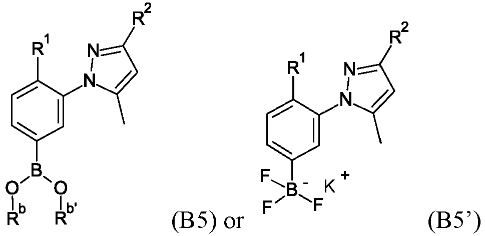
   with a compound of formula (B6) 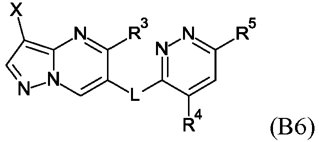
   in presence of a suitable solvent.

In the above process, R^{a} is alkyl, R^{b} and R^{b}' are independently selected from hydrogen and alkyl, or R^{b} and R^{b}' together with the oxygen atoms to which they are attached to form a five to six membered ring with the remaining ring members being carbon atoms optionally substituted with one or two alkyl substituents. X is halo, in particular bromo; L, A¹, A², R¹, R², R³, R⁴ and R⁵ are as described herein.

In the reaction of step (a) the reducing agent can be for example LiBH₄ or NaBH₄, optionally in the presence of LiCl.

In the reaction of step (a) the solvent can be for example MeOH optionally with a suitable cosolvent. The cosolvent can be for example THF, 1,4-dioxane, EtOH, dichloromethane or a mixture thereof.

Convenient conditions for step (a) can be between around -60 °C and around room temperature, in particular between around -40 °C and room temperature.

Conveniently, the conditions for step (a) are NaH₄ as reducing agent, MeOH as solvent with a suitable cosolvent and a reaction temperature of between around -40 °C and room temperature.

In the reaction of step (b) the Grignard reagent can be for example methylmagnesium bromide, methylmagesium chloride, ethylmagnesium bromide or ethylmagesium chloride.

In the reaction of step (b) the solvent can be for example THF or diethyl ether optionally with toluene as cosolvent.

Convenient conditions for step (b) can be between around -90 °C and -50 °C, in particular around -80 °C and around -60 °C, more particular around -70 °C.

Conveniently, the conditions for step (b) are methylmagesium bromide as reducing agent, THF as solvent and a reaction temperature of around -70 °C.

In the reaction of step (c) the base can be for example Cs₂CO₃, K₂CO₃ or K₃PO₄.

In the reaction of step (c) the catalyst can be for example a suitable Pd catalyst, in particular t-BuXPhos-Pd-G3 or [t-BuBrettPhos Pd(allyl)]OTf.

In the reaction of step (c) the solvent can be for example 1,4-dioxane, THF, DMA, DMF, NMP, toluene, xylene, water or a mixture thereof.

Convenient conditions for step (c) can be between around 50 °C and 120 °C, in particular around 70 °C and around 100 °C, more particular between around 80 °C and around 90 °C.

Conveniently, the conditions for step (c) are Cs₂CO₃ or K₂CO₃ as base, t-BuXPhos-Pd-G3 or [t-BuBrettPhos Pd(allyl)]OTf as catalyst and 1,4-dioxane as solvent at a reaction temperature of between around 80 °C and around 90 °C.

In the reaction of step (d) the base can be for example Cs₂CO₃ or K₂CO₃.

In the reaction of step (d) the catalyst can be for example Pd(dppf)Cl₂·CH₂Cl₂.

In the reaction of step (d) the solvent can be for example 1,4-dioxane, THF, DMF, DMA, NMP, toluene, H₂O or mixtures thereof.

Convenient conditions for step (d) can be between around 30 °C and 100 °C, in particular around 40 °C and around 90 °C, more particular between around 50 °C and around 80 °C.

Conveniently, the conditions for step (d) are Cs₂CO₃ or K₂CO₃ as base, t-BuXPhos-Pd-G3 or [t-BuBrettPhos Pd(allyl)]OTf as catalyst and 1,4-dioxane as solvent at a reaction temperature of between around 80 °C and around 90 °C.

The invention also relates in particular to:
A compound of formula (I) as describe herein or a pharmaceutically acceptable salt thereof, for use as therapeutically active substance;
A pharmaceutical composition comprising a compound of formula (I) as described herein or a pharmaceutically acceptable salt thereof, and a therapeutically inert carrier;
A compound of formula (I) as described herein or a pharmaceutically acceptable salt thereof, for use in the treatment or prophylaxis of rheumatoid arthritis, juvenile rheumatoid arthritis, non-alcoholic steatohepatitis (NASH), primary sclerosing cholangitis, giant cell vasculitis, inflammatory bowel diseases (IBD), atherosclerosis, type 2 diabetes or glomerulonephritis, in particular inflammatory bowel diseases (IBD); and

### Pharmaceutical Compositions

Another embodiment of the invention provides a pharmaceutical composition or medicament containing a compound of the invention and a therapeutically inert carrier, diluent or excipient, as well as a method of using the compounds of the invention to prepare such composition and medicament. In one example, the compound of formula (I) may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula (I) is formulated in an acetate buffer, at pH 5. In another embodiment, the compound of formula (I) is sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal, epidural and intranasal, and if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

The invention will now be illustrated by the following examples.

### Examples

### Abbreviations

- [t-BuBrettPhos Pd(allyl)]OTf: allyl(2-di-tert-butylphosphino-3,6-dimethoxy-2',4',6'-triisopropyl-1,1'-biphenyl)palladium(II) triflate (CAS # 1798782-15-6)
- ATP: adenosine triphosphate
- aq.: aqueous
- Boc: tert-butyloxycarbonyl
- CAS: chemical abstracts service
- DBU: 1,8-diazabicyclo[5.4.0]undec-7-ene
- DCM: dichloromethane
- DIBAL-H: diisobutylaluminium hydride
- DIPEA: *N,N*-diisopropylethylamine
- DMF: *N,N* -dimethylformamide
- DMSO: dimethyl sulfoxide
- dppf: 1,1'-ferrocenediyl-bis(diphenylphosphine)
- equiv.: equivalents
- ESI: electrospray ionization
- Et: ethyl
- EtOAc: ethyl acetate
- EtOH: ethanol
- FA: formic acid
- HATU: (1-[bis(dimethylamino)methylene]-1*H*-1,2,3-triazolo[4,5-b]pyridinium 3-oxide hexafluorophosphate
- HPLC: high pressure liquid chromatography
- LC-MS: high-performance liquid chromatography - mass spectrometry
- Me: methyl
- MeOH: methanol
- NIS: N-iodosuccinimide
- NMP: N-Methyl-2-pyrrolidone
- NMR: nuclear magnetic resonance
- PE: petroleum ether
- psi: pounds per square inch
- QToF: quadruploe time of flight
- RT: room temperature
- sat.: saturated
- t-BuXPhos-Pd-G3: [(2-Di-tert-butylphosphino-2',4',6'-triisopropyl-1,1'-biphenyl)-2-(2'-amino-1,1'-biphenyl)] palladium(II) methanesulfonate (CAS # 1447963-75-8)
- TEA: triethylamine
- Tf: triflyl
- TFA: trifluoroacetic acid
- THF: tetrahydrofuran
- TLC: thin layer chromatography
- TsOH: tosylic acid

### Example 1

### 1-[2-(1-Hydroxyethyl)-5-[6-[(6-methylpyridazin-3-yl)amino]imidazo[4,5-b]pyridin-3-yl]phenyl]-5-methyl-pyrazole-3-carbonitrile

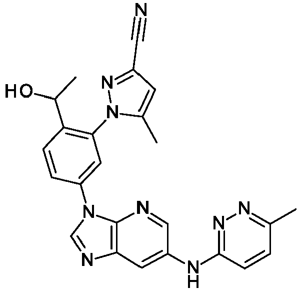

### Step 1: 1-(2-Fluoro-4-nitro-phenyl)ethanone

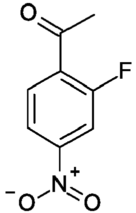

To a brown turbid solution of 4-bromo-3-fluoronitrobenzene (CAS 185331-69-5; 10.0 g, 45.45 mmol, 1.0 equiv.) in toluene (200 mL) was added tributyl(1-ethoxyvinyl)tin (CAS 20420-43-3; 24.6 g, 68.2 mmol, 1.5 equiv.), bis(triphenylphosphine)palladium(II) chloride (CAS 13965-03-2; 2.39 g, 3.41 mmol, 0.08 equiv.) The reaction mixture was stirred at 110 °C for 16 hours under a nitrogen atmosphere whereby it turned into a black solution. The mixture was cooled to RT, quenched with saturated aqueous potassium fluoride solution (300 ml) and stirred at room temperature for 16 hours. The aqueous phase was extracted with EtOAc. The biphasic mixture was filtered, the organic phase filtrate was separated, washed with brine, dried over sodium sulfate, filtered and concentrated. The crude product was purified by silica gel chromatography using a petroleum ether/ethylacetate gradient as eluent to give a mixture of the title compound and 1-(1-ethoxyvinyl)-2-fluoro-4-nitro-benzene as light brown oil which was dissolved in THF (90 mL) and 1 N aqueous HCl aqueous (90 mL). The mixture was stirred at RT for 6 hours, then poured into aquous saturated NaHCO₃ solution to bring the pH to around 8. The aqueous solution was extracted with EtOAc. The combined organics were washed with brine, dried over Na₂SO₄, filtered and concentrated. The crude product was purified by silica gel chromatography using a petroleum ether/ethyl acetate gradient as eluent to provide the title compound (6.4 g, 34.95 mmol, 77% yield) as yellow solid.

¹H NMR (400 MHz, CDCl₃-d) δ = 8.12 - 8.08 (m, 1H), 8.07 - 8.02 (m, 2H), 2.71 (d, J = 4.6 Hz, 3H).

### Step 2: 1-(2-Acetyl-5-nitro-phenyl)-5-methyl-pyrazole-3-carbonitrile

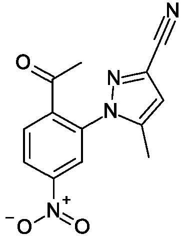

To a light brown clear solution of 5-methyl-1H-pyrazole-3-carbonitrile (CAS 38693-82-2; 4.12 g, 38.4 mmol, 1.1 equiv.) in DMSO (20 mL) was added 1-(2-fluoro-4-nitro-phenyl)ethanone (6.4 g, 34.95 mmol, 1.0 equiv.) and NaHCO₃ (5.87 g, 69.9 mmol, 2.0 equiv.). The brown suspension was stirred at 60 °C for 16 hours under N₂. Then the reaction was poured into water. The aqueous phase was extracted with EtOAc. The combined organic layers were washed with brine, dried over NaSO₄, filtered, concentrated under vacuum. The crude product was purified by prep-HPLC using a heptane/0.1% NH₃-solution in EtOH as eluent to provide the title compound (4.0 g, 14.8 mmol, 42% yield) as brown solid and the isomer 2-(2-acetyl-5-nitro-phenyl)-5-methylpyrazole-3-carbonitrile (800 mg, 2.96 mmol, 8% yield) as brown solid. LC-MS: m/z = 271.0 [M+H]⁺, ESI pos.

¹H NMR (400 MHz, CDCl₃-d) δ = 8.47 (dd, J = 2.1, 8.5 Hz, 1H), 8.26 (d, J = 2.1 Hz, 1H), 7.90 (d, J = 8.6 Hz, 1H), 6.67 (s, 1H), 2.31 (s, 3H), 2.27 (s, 3H).

### Step 3: 1-(2-Acetyl-5-amino-phenyl)-5-methyl-pyrazole-3-carbonitrile

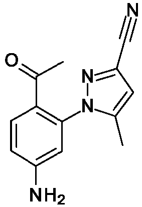

To the solution of 1-(2-acetyl-5-nitro-phenyl)-5-methyl-pyrazole-3-carbonitrile (4.0 g, 14.8 mmol, 1.0 equiv.) in EtOH (30 mL) was added Fe (4.13 g, 74.0 mmol, 5.0 equiv.), NH₄Cl (8.0 g, 149.6 mmol, 10.1 equiv.) and water (10 mL). The reaction mixture was stirred under a nitrogen atmosphere at 50 °C for 16 hours. The color of the mixture changed from yellow to brown. The suspension was filtered through a pad of silica gel and filter cake was washed with EtOH. The combined filtrates were concentrated. The crude product was purified by silica gel chromatography to give the title compound (3.5 g, 14.7 mmol, 98% yield) as brown solid. LC-MS: m/z = 241.2[M+H]⁺, ESI pos.
¹H NMR (400 MHz, MeOH-d₄) δ = 7.82 (d, J = 8.7 Hz, 1H), 6.81 (dd, J = 2.3, 8.7 Hz, 1H), 6.71 (d, J = 0.6 Hz, 1H), 6.52 (d, J = 2.3 Hz, 1H), 2.19 (s, 3H), 2.14 (s, 3H).

### Step 4: 1-[2-Acetyl-5-[(3,5-dinitro-2-pyridyl)amino]phenyl]-5-methyl-pyrazole-3-carbonitrile

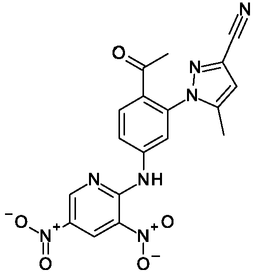

A solution of 2-chloro-3,5-dinitropyridine (CAS 2578-45-2; 2.91 g, 14.3 mmol, 0.98 equiv.) and 1-(2-acetyl-5-amino-phenyl)-5-methyl-pyrazole-3-carbonitrile (3.5 g, 14.57 mmol, 1.0 equiv.) in MeOH (60 mL) was stirred at 50 °C for 16 hours. The resulting yellow suspension was concentrated. The crude product was purified by column chromatography using a petroleum ether/ethyl acetate gradient as eluent to yield the product as yellow solid. It was triturated with MeOH (20 mL) at 25 °C for 30 minutes. The suspension was filtrated. The solids were washed with EtOAc. The combined filtrates were concentrated to dryness to give the title compound (4.2 g, 10.3 mmol, 70% yield) as yellow solid. LC-MS: m/z = 408.1[M+H]⁺ ESI pos
¹H NMR (400 MHz, CDCl₃-d) δ = 10.79 (s, 1H), 9.39 (s, 2H), 7.99 (d, J = 2.0 Hz, 1H), 7.94 - 7.91 (m, 1H), 7.89 - 7.86 (m, 1H), 6.66 (s, 1H), 2.27 (s, 3H), 2.20 (s, 3H).

### Step 5: 1-[2-Acetyl-5-[(3,5-diamino-2-pyridyl)amino]phenyl]-5-methyl-pyrazole-3-carbonitrile

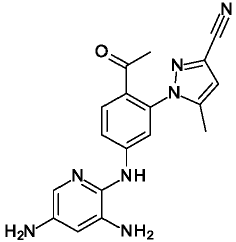

To a solution of 1-[2-acetyl-5-[(3,5-dinitro-2-pyridyl)amino]phenyl]-5-methyl-pyrazole-3-carbonitrile (4.2 g, 10.31 mmol, 1.0 equiv.) in EtOH (30 mL) was added Fe (2.88 g, 51.6 mmol, 5.0 equiv.), NH₄Cl (5.52 g, 103.1 mmol, 10.0 equiv.) and water (10 mL). The reaction mixture was stirred under a nitrogen atmosphere at 50 °C for 16 hours. The resulting suspension was filtered through a pad of silica gel and the residue was washed with EtOH. The combined filtrates were concentrated. The remaining solid was washed with water and extraced with EtOAc and subsequently with DCM/MeOH (10:1). The combined organic layers were dried over Na₂SO₄, filtered and concentrated to give the title compound (3.5 g, 10.1 mmol, 98% yield) as dark green solid. LC-MS: m/z = 348.1[M+H]⁺ ESI pos
¹H NMR (400 MHz, MeOH-d₄) δ = 7.88 (d, J = 8.8 Hz, 1H), 7.24 (d, J = 2.5 Hz, 1H), 7.01 (dd, J = 2.4, 8.8 Hz, 1H), 6.71 - 6.70 (m, 2H), 6.60 (d, J = 2.5 Hz, 1H), 2.20 (s, 3H), 2.16 (s, 3H).

### Step 6: 1-[2-Acetyl-5-(6-aminoimidazo[4,5-b]pyridin-3-yl)phenyl]-5-methyl-pyrazole-3-carbonitrile

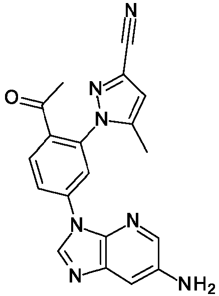

A mixture of 1-[2-acetyl-5-[(3,5-diamino-2-pyridyl)amino]phenyl]-5-methyl-pyrazole-3-carbonitrile (3.5 g, 10.1 mmol, 1.0 equiv.) in EtOH (100 mL) was treated with trimethoxymethane (11.0 mL, 100.4 mmol, 10.0 equiv.) and with TsOH (155.0 mg, 1.01 mmol, 0.1 equiv.) and heated at 120 °C for 4 hours. The reaction mixture was concentrated. The residue was dissolved in THF (60mL) and 1N HCl (60mL) and stirred at RT for 4 hours. The reaction mixture was quenched with NaHCO₃ solution and extracted with EtOAc. The combined organic layers were dried over Na₂SO₄, filtered and concentrated. The residue was triturated with MeOH and EtOAc at RT for 30 minutes. The mixture was filtered. The solid was washed with petroleum ether and dried to give the title compound (2.8 g, 7.84 mmol, 81% yield) as brown solid. LC-MS: m/z = 358.1 [M+H]⁺ ESI pos.
¹H NMR (400 MHz, DMSO-d6) δ = 8.92 (s, 1H), 8.52 (dd, J = 2.2, 8.6 Hz, 1H), 8.34 (d, J = 2.1 Hz, 1H), 8.15 (d, J = 8.5 Hz, 1H), 7.91 (d, J = 2.4 Hz, 1H), 7.30 (d, J = 2.4 Hz, 1H), 7.03 (d, J = 0.8 Hz, 1H), 5.27 (s, 2H), 2.29 (s, 3H), 2.23 (s, 3H).

### Step 7: 1-[2-Acetyl-5-(6-bromoimidazo[4,5-b]pyridin-3-yl)phenyl]-5-methyl-pyrazole-3-carbonitrile

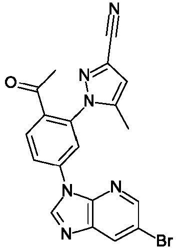

To a suspension of copper (I) dibromide (2.43 g, 10.9 mmol, 1.6 equiv.) in acetonitrile (30 mL) was added isoamyl nitrite (1.63 g, 13.9 mmol, 2.0 equiv.). The green-brown suspension was heated up to 65 °C under a nitrogen atmosphere. A suspension of 1-[2-acetyl-5-(6-aminoimidazo[4,5-b]pyridin-3-yl)phenyl]-5-methyl-pyrazole-3-carbonitrile (2.7 g, 6.8 mmol, 1.0 equiv.) in acetonitrile (30 mL) was added to the reaction mixture which turned black. The reaction was stirred for 10 minutes, then quenched with water and extracted with EtOAc. The combined organic layers were dried over Na₂SO₄, filtered and concentrated. The residue was triturated with MeOH (10 mL), petroleum ether (30 mL) and DCM (5 mL) at 25 °C for 30 minutes. The mixture was filtered. The solids were dried to give a first crop of the title compound (853.8 mg, 2.03 mmol, 28% yield) as brown solid. The filtrate was concentrated and purified by silica gel chromatography using a hexane/EtOH + 0.1% formic acid gradient to obtain a second crop of title compound (596 mg, 1.4 mmol, 19% yield) as light brown solid. LC-MS: m/z = 421.0 [M+H]⁺, ESI pos.

### Step 8: 1-[2-Acetyl-5-[6-[(6-methylpyridazin-3-yl)amino]imidazo[4,5-b]pyridin-3-yl]phenyl]-5-methyl-pyrazole-3-carbonitrile

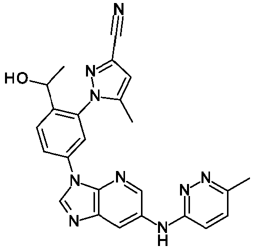

A suspension of 1-[2-acetyl-5-(6-bromoimidazo[4,5-b]pyridin-3-yl)phenyl]-5-methyl-pyrazole-3-carbonitrile (50 mg, 0.12 mmol, 1.0 equiv.), (6-methylpyridazin-3-yl)amine (CAS 18591-82-7; 25.9 mg, 0.237 mmol, 2.0 equiv.) and Cs₂CO₃ (116.0 mg, 0.356 mmol, 3.0 equiv.) at RT in 1,4-dioxane, extra dry (1.67 mL) was flushed with argon for 5 minutes. Then [t-BuBrettPhos Pd(allyl)]OTf (9.27 mg, 0.012 mmol, 0.1 equiv.) was added, the vial was locked and the mixture was then heated to 80 °C and stirring was continued for 2 hours. The reaction mixture was cooled to RT. The crude product was purified by silica gel chromatography using a dichloromethane/MeOH gradient as eluent to provide the title compound (55 mg, 82% yield) as off-white solid. LC-MS: m/z = 449.2 [M+H]⁺, ESI pos.

### Step 9: 1-[2-(1-Hydroxyethyl)-5-[6-[(6-methylpyridazin-3-yl)amino]imidazo[4,5-b]pyridin-3-yl]phenyl]-5-methyl-pyrazole-3-carbonitrile

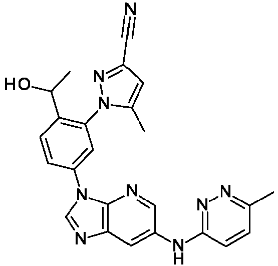

To a mixture of 1-[2-acetyl-5-[6-[(6-methylpyridazin-3-yl)amino]imidazo[4,5-b]pyridin-3-yl]phenyl]-5-methyl-pyrazole-3-carbonitrile (54.7 mg, 0.122 mmol, 1.0 equiv.) in methanol (1.0 mL) and dry tetrahydrofuran (1.0 mL) was added NaBH₄ (6.91 mg, 0.18 mmol, 1.5 equiv.) at 0 °C. The mixture was stirred at 0 °C for 15 minutes, then warmed to RT and stirred for 2 hours. The mixture was diluted with dry DMF (0.52 mL), cooled to 0 °C and treated with NaBH₄ (6.91 mg, 0.183 mmol, 1.5 equiv.). The mixture was stirred at 0 °C for 30 minutes, then quenched with saturated aqueous NH₄Cl solution and extracted with DCM. The organic layer was washed with brine, dried over MgSO₄ filtered and concentrated. The crude product was purified by silica gel chromatography using a DCM/MeOH gradient as eluent to provide the title compound (29 mg, 51% yield) as light yellow solid. LC-MS: m/z = 452.4 [M+H]⁺, ESI pos.

### Example 2

### 1-[2-(1-Hydroxyethyl)-5-[6-[(6-methylpyridazin-3-yl)amino]pyrazolo[1,5-a]pyrimidin-3-yl]phenyl]-5-methyl-pyrazole-3-carbonitrile

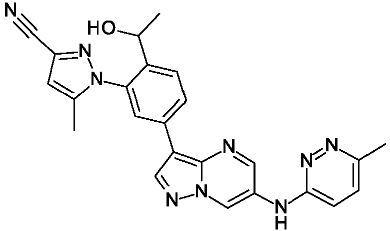

### Step 1: 1-(2-Acetyl-5-bromo-phenyl)-5-methyl-pyrazole-3-carbonitrile

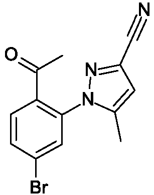

A solution of 1-(4-bromo-2-fluoro-phenyl)ethanone (CAS 625446-22-2; 1 g, 4.61 mmol, 1.0 equiv.) in DMF (15.9 mL) was treated with K₂CO₃ (955 mg, 6.9 mmol, 1.5 equiv.) and 5-methyl-1H-pyrazole-3-carbonitrile (592 mg, 5.53 mmol, 1.2 equiv.). The reaction mixture was stirred at 100 °C overnight. The reaction mixture was poured into water and extracted with ethyl acetate. The organic layers were washed with brine, combined, dried over with MgSO₄, filtered and concentrated. The crude product was purified by silica gel chromatography to provide the title compound (812 mg, 57% yield) as light brown solid. LC-MS: m/z = 304.4 [M+H]⁺, ESI pos.

### Step 2: 1-[2-Acetyl-5-(4,4,5,5-tetramethyl-1,3,2-dioxaborolan-2-yl)phenyl]-5-methyl-pyrazole-3-carbonitrile

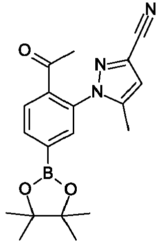

To a light yellow solution of 1-(2-acetyl-5-bromo-phenyl)-5-methyl-pyrazole-3-carbonitrile (785 mg, 2.58 mmol, 1 equiv.) in DMSO (10 mL) was added bis(pinacolato)diboron (CAS 73183-34-3; 786 mg, 3.1 mmol, 1.2 equiv.) and potassium acetate (760 mg, 7.7 mmol, 3 equiv.) followed by Pd(dppf)Cl₂ (189 mg, 0.26 mmol, 0.1 equiv.). The reaction mixture was stirred at 90 °C for 2 hours. The reaction mixture was poured into water and extracted two times with ethyl acetate. The organic layers were back-extracted with sat NaCl, dried over MgSO₄ and concentrated. The crude material was purified by silica gel chromatography to afford the title compound (908 mg, quant.) as light brown solid. LC-MS: m/z = 270.1 [M-pinacol+H]⁺, ESI pos.

### Step 3: 1-[2-Acetyl-5-(6-bromopyrazolo[1,5-a]pyrimidin-3-yl)phenyl]-5-methyl-pyrazole-3-carbonitrile

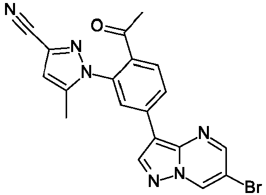

A solution of 1-[2-acetyl-5-(4,4,5,5-tetramethyl-1,3,2-dioxaborolan-2-yl)phenyl]-5-methylpyrazole-3-carbonitrile (900 mg, 2.6 mmol, 1.0 equiv.) in 1,4-dioxane (12 mL) was treated with 6-bromo-3-iodo-pyrazolo[1,5-a]pyrimidine (CAS 1109284-33-4; 996 mg, 3.1 mmol, 1.2 equiv.), a solution of K₂CO₃ (885 mg, 6.4 mmol, 2.5 equiv.) solved in water (3 mL) and Pd(dppf)Cl₂·CH₂Cl₂ complex (424 mg, 0.51 mmol, 0.2 equiv.). The reaction mixture was stirred at 50 °C for 90 minutes, then poured into water and extracted twice with ethyl acetate. The combined organic layers were dried over MgSO₄, filtered and concentrated. The crude product was purified by silica gel chromatography using a heptane/ethyl acetate gradient as eluent to provide the title compound (711 mg, 60% purity, 39% yield) as brown solid. LC-MS: m/z = 423.1 [M+H]⁺, ESI pos.

### Step 4: 1-[2-Acetyl-5-[6-[(6-methylpyridazin-3-yl)amino]pyrazolo[1,5-a]pyrimidin-3-yl]phenyl]-5-methyl-pyrazole-3-carbonitrile

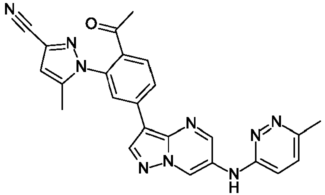

A solution of 1-[2-acetyl-5-(6-bromopyrazolo[1,5-a]pyrimidin-3-yl)phenyl]-5-methyl-pyrazole-3-carbonitrile (100 mg, 0.24 mmol, 1.0 equiv.) in 1,4-dioxane (2 mL) was treated with (6-methylpyridazin-3-yl)amine (51.8 mg, 0.48 mmol, 2.0 equiv.) and Cs₂CO₃ (232 mg, 0.71 mmol, 3.0 equiv.) at RT. The reaction mixture was degassed and flushed with argon, before [t-BuBrettPhos Pd(allyl)]OTf (18.6 mg, 0.024 mmol, 0.1 equiv.) was added. The mixture was stirred for 2 hours at 80 °C, then diluted with water and extracted two times with EtOAc. The organic layers were dried over MgSO₄ and concentrated. The crude product was purified by silica gel chromatography using a DCM/methanol gradient as eluent to provide the title compound (28.7 mg, 24% yield) as yellow solid. LC-MS: m/z = 450.3 [M+H]⁺, ESI pos.

### Step 5: 1-[2-(1-Hydroxyethyl)-5-[6-[(6-methylpyridazin-3-yl)amino]pyrazolo[1,5-a]pyrimidin-3-yl]phenyl]-5-methyl-pyrazole-3-carbonitrile

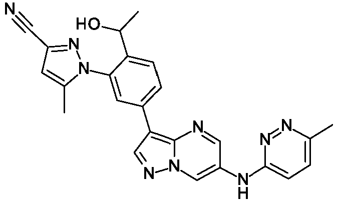

A solution of 1-[2-acetyl-5-[6-[(6-methylpyridazin-3-yl)amino]pyrazolo[1,5-a]pyrimidin-3-yl]phenyl]-5-methyl-pyrazole-3-carbonitrile (28.7 mg, 0.06 mmol, 1.0 equiv.) in isopropanol (1.5 mL) and THF (1.5 mL) was treated with NaBH₄ (4.8 mg, 0.13 mmol, 2.0 equiv.) and then stirred at RT for 1 hour. The reaction mixture was diluted with water and extracted with ethyl acetate. The organic layers were dried over MgSO₄ and concentrated. The crude product was purified by silica gel chromatography using a DCM/MeOH gradient to give the title compound (10.5 mg, 36% yield) as yellow solid. LC-MS: m/z = 452.3 [M+H]⁺, ESI pos.

### Example 3

### 1-[2-[3-(Difluoromethyl)-5-methyl-pyrazol-1-yl]-4-[6-(pyridazin-3-ylamino)imidazo[4,5-b]pyridin-3-yl]phenyl]ethanol

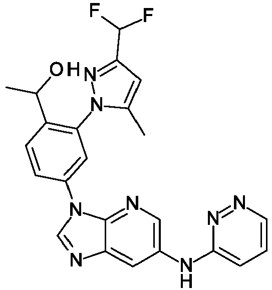

### Step 1: 1-(2-Fluoro-4-nitro-phenyl)ethanone

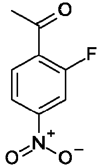

To a brown turbid solution of 4-bromo-3-fluoronitrobenzene (10.0 g, 45.5 mmol, 1.0 equiv.) in Toluene (200 mL) was added tributyl(1-ethoxyvinyl)tin (24.62 g, 68.2 mmol, 1.5 equiv.) and bis(triphenylphosphine)palladium(II) chloride (2.39 g, 3.4 mmol, 0.08 equiv.). The reaction was stirred at 110 °C for 16 hours under a nitrogen atmosphere, turning into a black solution. The mixture reaction was cooled to RT, quenched with saturated aqueous potassium fluoride solution (300 ml), stirred at RT for 16 hours and extracted with ethyl acetate. The mixture was filtered. The organic phase of the filtrate was separated, washed with brine, dried over sodium sulfate, filtered and concentrated. The residue was purified by silica gel chromatography using a petroleum ether/ethyl acetate gradient to give a mixture of 1-(1-ethoxyvinyl)-2-fluoro-4-nitrobenzene and desired product 1-(2-fluoro-4-nitro-phenyl)ethanone (10 g) as light brown oil. The mixture was dissolved in THF 90 mL and 1 N HCl aqueous 90 mL and stirred at 30 °C (room temperature) for 2 hours. The mixture was poured into sat. NaHCO₃ aqueous (300 mL, pH of aqeous phase ca. 8). The aqueous solution was extracted with EtOAc. The combined organics were washed with brine, dried over Na₂SO₄, filtered and concentrated. The crude product was purified by silica gel chromatography using a petroleum ether/ethyl acetate gradient as eluent to give the title compound (5.0 g, 27.3 mmol, 60% yield) as yellow oil.

¹H NMR (400 MHz, CDCl₃-d) δ = 8.13 - 8.10 (m, 1H), 8.09 - 8.04 (m, 2H), 2.73 (d, J = 4.8 Hz, 3H).

### Step 2: ]-(2-(3-(Difluoromethyl)-5-methyl-1H-pyrazol-1-yl)-4-nitrophenyl)ethan-1-one

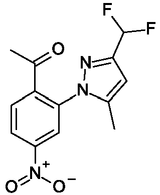

To a solution of 1-(2-fluoro-4-nitro-phenyl)ethanone (CAS 866579-96-6; 2.0 g, 10.9 mmol, 1 equiv.) in DMSO (30 mL) was added 3-(difluoromethyl)-5-methyl-1H-pyrazole (CAS 934759-09-8; 1.44 g, 10.9 mmol, 1 equiv.), followed by NaHCO₃ (1.83 g, 21.8 mmol, 2 eq). The reaction mixture was stirred at 80 °C for 12 hours, then diluted with H₂O and extracted with ethyl acetate. The organic phase was washed with brine, dried over anhydrous Na₂SO₄, filtered and concentrated. The crude product was purified by silica gel chromatography using an ethyl acetate/petroleum ether gradient as eluent and a second time by prep-HPLC using a 0.225% aqueous formic acid/acetonitrile gradient as eluent to give title compound in mixture with its isomer (400 mg, 12% yield; mixture with isomer 1-(2-(5-(difluoromethyl)-3-methyl-1H-pyrazol-1-yl)-4-nitrophenyl)ethan-1-one) as red solid. LC-MS: m/z = 296.1[M+H]⁺ ESI pos.

### Step 3: 1-(4-Amino-2-(3-(difluoromethyl)-5-methyl-1H-pyrazol-1-yl)phenyl)ethan-1-one

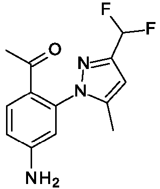

To a solution of 1-[2-[3-(difluoromethyl)-5-methyl-pyrazol-1-yl]-4-nitro-phenyl]ethanone (650 mg, 2.2 mmol, 1 equiv.; mixture with isomer 1-(2-(5-(difluoromethyl)-3-methyl-1H-pyrazol-1-yl)-4-nitrophenyl)ethan-1-one) in ethanol (9 mL) and water (3 mL) was added Fe (616 mg, 11.0 mmol, 5 equiv.) and NH₄Cl (1.17 g, 22.0 mmol, 10 equiv.). The reaction mixture was stirred at 50 °C for 3 hours. The reaction was filtered, and the filtrate was concentrated to give the title compound (mixture with isomer 1-(4-amino-2-(5-(difluoromethyl)-3-methyl-1H-pyrazol-1-yl)phenyl)ethan-1-one) as crude product which was used in the next step without further purification.

### Step 4: 1-(4-Bromo-2-(3-(difluoromethyl)-5-methyl-1H-pyrazol-1-yl)phenyl)ethan-1-one

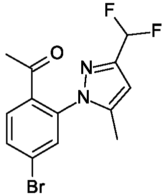

A mixture of copper(I) bromide (240.6 mg, 1.7 mmol, 1 equiv.) and isoamyl nitrite (397.5 mg, 3.39 mmol, 2 equiv.) in acetonitrile (10 mL) was stirred at 65 °C for 30 minutes until the mixture turned black. Then, 1-[4-amino-2-[3-(difluoromethyl)-5-methyl-pyrazol-1-yl]phenyl]ethanone (450 mg, 1.7 mmol, 1 equiv.; mixture of two isomers) were added. The reaction mixture was stirred at 65 °C for 30 minutes. The crude product was purified by prep-TLC using petrol ether/ethyl acetate 3:1 as eluent to give the title compound (250 mg, 0.76 mmol, 45% yield; mixture with isomer 1-[4-bromo-2-[5-(difluoromethyl)-3-methyl-pyrazol-1-yl]phenyl]ethanone) as white solid. LC-MS: m/z = 328.9[M+H]⁺ ESI pos.

¹H NMR (400 MHz, CDCl₃-d) δ = 7.67 - 7.61 (m, 1H), 7.59 - 7.53 (m, 1H), 7.48 (d, *J* = 1.7 Hz, 1H), 6.73 - 6.43 (m, 1H), 6.40 (s, 1H), 2.16 (s, 3H), 1.88 (s, 3H).

### Step 5: (4-Acetyl-3-(3-(difluoromethyl)-5-methyl-1H-pyrazol-1-yl)phenyl)boronic acid

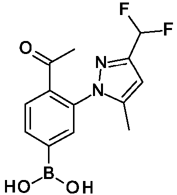

To a solution of 1-[4-bromo-2-[3-(difluoromethyl)-5-methyl-pyrazol-1-yl]phenyl]ethanone (250 mg, 0.76 mmol, 1 equiv.; mixture with isomer 1-[4-bromo-2-[5-(difluoromethyl)-3-methyl-pyrazol-1-yl]phenyl]ethanone) in 1,4-dioxane (5 mL) was added bis(pinacolato)diboron (289 mg, 1.14 mmol, 1.5 equiv.), Pd(dppf)Cl₂·CH₂Cl₂ (53.5 mg, 0.08 mmol, 0.1 equiv.) and KOAc (223.3 mg, 2.28 mmol, 3 equiv.). The mixture was stirred at 90 °C for 3 hours under a nitrogen atmosphere, then concentrated. The crude product was purified by prep-HPLC using a 0.225% aqueous formic acid/acetonitrile gradient as eluent to give the title compound (80 mg, 0.27 mmol, 36% yield) as white solid. LC-MS: m/z = 295.0[M+H]⁺, ESI pos.

### Step 6: 1-(4-(6-Bromo-3H-imidazo[4,5-b]pyridin-3-yl)-2-(3-(difluoromethyl)-5-methyl-1H-pyrazol-1-yl)phenyl)ethan-1-one

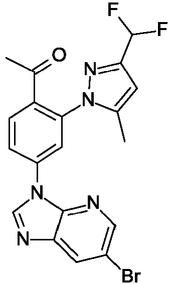

To a solution of 6-bromo-3H-imidazo[4,5-b]pyridine (CAS 28279-49-4; 45 mg, 0.23 mmol, 1 equiv.) in MeOH (2 mL) was added [4-acetyl-3-[3-(difluoromethyl)-5-methyl-pyrazol-1-yl]phenyl]boronic acid (73.5 mg, 0.25 mmol, 1.1 equiv.) and Cu(OAc)₂ (8.22 mg, 0.05 mmol, 0.2 equiv.). The reaction mixure was stirred at 50 °C for 12 hours under an O₂ atmosphere, then concentrated. The crude product was purified to give the title compound (40 mg, 0.09 mmol, 36% yield) as white solid. LC-MS: m/z = 448.0[M+H]⁺ ESI pos.; 446.0[M+H]⁺ ESI pos.

### Step 7: 1-(2-(3-(Difluoromethyl)-5-methyl-1H-pyrazol-1-yl)-4-(6-(pyridazin-3-ylamino)-3H-imidazo[4,5-b]pyridin-3-yl)phenyl)ethan-1-one

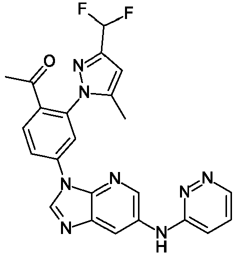

To a solution of 1-[4-(6-bromoimidazo[4,5-b]pyridin-3-yl)-2-[3-(difluoromethyl)-5-methyl-pyrazol-1-yl]phenyl]ethanone (30.0 mg, 0.07 mmol, 1 equiv.) in 1,4-dioxane (2 mL) was added 3-aminopyridazine (12.8 mg, 0.130 mmol, 2 equiv.), followed by [t-BuBrettPhos Pd(allyl)]OTf (5.25 mg, 0.01 mmol, 0.1 equiv.) Cs₂CO₃ (65.7 mg, 0.2 mmol, 3 equiv.). The reaction mixture was stirred at 90 °C for 4 hours under a nitrogen atmosphere, then diluted with ethyl acetate and filtered. The filtrate was concentrated. The crude product was purified by TLC using dichloroemethane/MeOH (10:1) as eluent to give the title compound (20 mg, 0.04 mmol, 65% yield) as yellow solid. LC-MS: m/z = 461.2[M+H]⁺, ESI pos.

¹H NMR (400 MHz, MeOH-d₄) δ = 8.91 (s, 1H), 8.77 (d, *J =* 2.3 Hz, 1H), 8.68 (d, *J =* 2.3 Hz, 1H), 8.63 (d, *J =* 3.9 Hz, 1H), 8.36 - 8.29 (m, 2H), 8.04 (d, *J =* 8.4 Hz, 1H), 7.49 (dd, *J =* 4.6, 9.1 Hz, 1H), 7.20 (dd, *J =* 1.1, 9.0 Hz, 1H), 6.88 - 6.60 (t, 1H), 6.58 (s, 1H), 2.40 (s, 3H), 2.08 (s, 3H).

### Step 8: 1-(2-(3-(Difluoromethyl)-5-methyl-1H-pyrazol-1-yl)-4-(6-(pyridazin-3-ylamino)-3H-imidazo[4,5-b]pyridin-3-yl)phenyl)ethan-1-ol

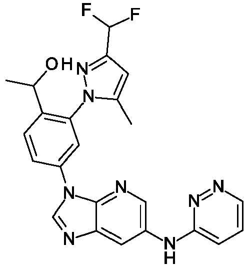

A solution of 1-[2-[3-(difluoromethyl)-5-methyl-pyrazol-1-yl]-4-[6-(pyridazin-3-ylamino)-imidazo[4,5-b]pyridin-3-yl]phenyl]ethanone (20.0 mg, 0.04 mmol, 1 equiv.) in methanol (1 mL) and DCM (1 mL) was cooled to -10 °C. A precipitate was formed, therefore DMF (1 mL) was added. The reaction mixture turned to a colorless solution and was then treated with NaBH₄ (4.93 mg, 0.130 mmol, 3 equiv.) at -10 °C. The reaction mixture was stirred at -10 °C for 30 minutes, then quenched with saturated NH₄Cl and extracted with DCM. The organic phases was washed with brine, dried over anhydrous Na₂SO₄, filtered and concentrated. The crude product was purified by prep-HPLC using a 0.225% aqueous formic acid/acetonitrile gradient as eluent. The product-containing fractions were concentrated and freeze-dried to give the title compound (8.9 mg, 0.020 mmol, 43% yield) as white solid. LC-MS: m/z = 463.1[M+H]⁺ ESI pos.

¹H NMR (400 MHz, MeOH-d₄) δ = 8.82 (s, 1H), 8.77 (d, *J =* 2.2 Hz, 1H), 8.70 - 8.63 (m, 2H), 8.21 (dd, *J =* 1.9, 8.5 Hz, 1H), 8.06 - 8.01 (m, 2H), 7.51 (dd, *J =* 4.6, 8.9 Hz, 1H), 7.22 (dd, *J =* 1.2, 9.2 Hz, 1H), 6.95 - 6.62 (m, 1H), 6.56 (s, 1H), 4.65 - 4.51 (m, 1H), 2.33 (s, 3H), 1.37 (d, *J =* 6.4 Hz, 3H).

### Example 4

### 3-[[3-[4-cyano-3-[3-(difluoromethoxy)-5-methyl-pyrazol-1-yl]phenyl]-5-methoxy-imidazo[4,5-b]pyridin-6-yl]amino]-N,N,6-trimethyl-pyridazine-4-carboxamide

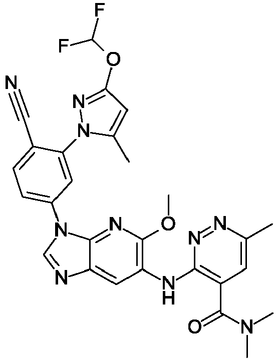

### Step 1: tert-Butyl 3-hydroxy-5-methyl-pyrazole-1-carboxylate

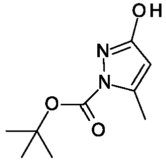

To a solution of 5-methyl-1H-pyrazol-3-ol (10.0 g, 101.9 mmol, 1.0 equiv.) in dichloromethane (100 mL) was added di-t-butyldicarbonate (23.44 mL, 101.9 mmol, 1.0 equiv.) and triethylamine (15.6 mL, 112.1 mmol, 1.1 equiv.). The reaction mixture was stirred at 25 °C for 18 hours, then was poured into water and extracted with dichloromethane. The organic layers were washed with brine and dried over Na₂SO₄, then concentrated. The crude product was triturated with petroleum ether (50 ml), filtered and dried to give the title compund (10.0 g, 50.5 mmol, 49% yield) as white solid. LC-MS: m/z = 143.0 [M+H-C4H8]⁺ ESI pos.

¹H NMR (400 MHz, CDCl₃-d) δ = 12.35 - 12.01 (m, 1H), 5.68 (d, J = 0.7 Hz, 1H), 2.48 (d, J = 0.6 Hz, 3H), 1.64 (s, 9H).

### Step 2: tert-Butyl 3-(difluoromethoxy)-5-methyl-pyrazole-1-carboxylate

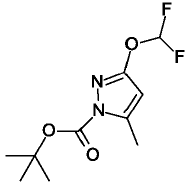

To a white suspension of sodium chlorodifluoroacetate (12.7 g, 83.2 mmol, 1.1 equiv.) and tert-butyl 3-hydroxy-5-methyl-pyrazole-1-carboxylate (15.0 g, 75.67 mmol, 1.0 equiv.) in acetonitrile (250 mL) was added Cs₂CO₃ (49.3 g, 151.35 mmol, 2.0 equiv.). The reaction mixture was stirred at 80 °C for 12 hours, then cooled to RT and filtered. The filtrate was concentrated. The crude product was purified by silica gel chromatography using a petroleum ether/ethyl acetate gradient to the title compound (10.0 g, 40.3 mmol, 53% yield) as colorless oil. LC-MS: m/z = 193.1 [M+H-C4H8]⁺ ESI pos.

¹H NMR (400 MHz, CDCl₃-d) δ = 7.06 (t, J = 72.6 Hz, 1H), 5.85 (s, 1H), 2.51 (s, 3H), 1.65 (s, 10H).

### Step 3: 3-(Difluoromethoxy)-5-methyl-1H-pyrazole hydrochloride

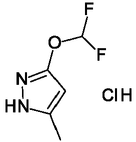

To a solution of tert-butyl 3-(difluoromethoxy)-5-methyl-pyrazole-1-carboxylate (10.0 g, 40.3 mmol, 1.0 equiv.) in DCM (30 mL) was added HCl in dioxane (30.0 mL, 120.0 mmol, 3.0 equiv.). The reaction mixture was stirred at 25 °C for 4 hours. The solution was concentrated and the crude product directly used in the next step without purification. LC-MS: m/z = 149.0 [M+H]⁺ ESI pos.

¹H NMR (400 MHz, CDCl₃-d) δ = 12.92 (br s, 2H), 6.71 (t, J = 71.2 Hz, 1H), 5.94 (s, 1H), 2.47 (s, 3H).

### Step 4: 2-[3-(Difluoromethoxy)-5-methyl-pyrazol-1-yl]-4-nitro-benzonitrile

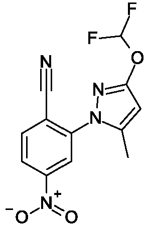

To a light colorless clear solution of 2-fluoro-4-nitrobenzonitrile (3.1 g, 18.6 mmol, 1.2 equiv.) and 3-(difluoromethoxy)-5-methyl-1H-pyrazole (2.3 g, 15.5 mmol, 1.0 equiv.) in DMSO (100 mL) was added K₂CO₃ (4.3 g, 31.1 mmol, 2.0 equiv.). Then the yellow suspension was stirred at 50 °C for 16 hours. After the mixture had cooled to room temperature, it was poured into water and extracted with ethyl acetate. The combined organics were washed with brine, dried over NaSO₄, filtered and concentrated. The crude product was purified by silica gel chromatography using a petroleum ether/ethyl acetate gradient to obtain the title compund (3.3 g, 11.2 mmol, 72% yield) as white solid. LC-MS: m/z = 295.1 [M+H]⁺, ESI pos.

¹H NMR (400 MHz, CDCl₃-d) δ = 8.44 - 8.31 (m, 2H), 8.04 (d, J = 8.4 Hz, 1H), 6.98 (t, J = 72.7 Hz, 1H), 6.03 (s, 1H), 2.39 (s, 3H).

### Step 5: 4-Amino-2-[3-(difluoromethoxy)-5-methyl-pyrazol-1-yl]benzonitrile

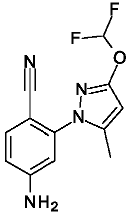

To a solution of 2-[3-(difluoromethoxy)-5-methyl-pyrazol-1-yl]-4-nitro-benzonitrile (3.3 g, 11.2 mmol, 1.0 equiv.) in EtOH (100 mL) was added Fe (3.13 g, 56.1 mmol, 5.0 equiv.), NH₄Cl (6.0 g, 112.2 mmol, 10.0 equiv.) and water (50 mL). The reaction mixture was stirred under a nitrogen atmosphere at 50 °C for 16 hours. Then, the mixture was filtered through a pad of celite while hot. The filtrate was concentrated. Water (50 mL) was added to the crude product. The aqueous was extracted with ethyl acetate. The combined organics were washed with brine, dried over Na₂SO₄, filtered and concentrated. The crude product was purified by silica gel chromatography using a petroleum ether/ethyl acetate eluent to give the title compound (2.05 g, 69% yield) as white solid. LC-MS: m/z = 265.0 [M+H]⁺, ESI pos.

¹H NMR (400 MHz, DMSO-d6) δ = 7.55 (d, J = 8.6 Hz, 1H), 7.29 (t, J = 73.2 Hz, 1H), 6.71 (dd, J = 2.2, 8.6 Hz, 1H), 6.62 (d, J = 2.2 Hz, 1H), 6.53 (s, 2H), 6.10 (s, 1H), 2.20 (s, 3H).

### Step 6: 4-[(5-Bromo-6-methoxy-3-nitro-2-pyridyl)amino]-2-[3-(difluoromethoxy)-5-methyl-pyrazol-1-yl]benzonitrile

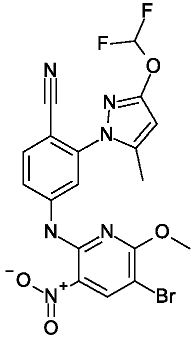

To a orange solution of 4-amino-2-[3-(difluoromethoxy)-5-methyl-pyrazol-1-yl]benzonitrile (1.0 g, 3.78 mmol, 1 equiv.) and 5-bromo-2-fluoro-6-methoxy-3-nitro-pyridine (1.04 g, 4.16 mmol, 1.1 equiv.) in THF (30 mL) was added lithium bis(trimethylsilyl)amide (11.35 mL, 11.35 mmol, 3 equiv.) dropwise at 0 °C under a nitrogen atmosphere. The mixture was stirred at 0 °C for 1 hour. The red reaction mixture was poured into water. The aqueous solution was extracted with ethyl acetate. The combined organic layers were washed with brine, dried over Na₂SO₄, filtered and concentrated. The crude product was purified by prep-TLC using petroleum ether/ethyl acetate 3:1 to give the title compound (1.8 g, 3.63 mmol, 96% yield) as brown solid. LC-MS: m/z = 494.8/496.8 [M+H]⁺, ESI pos.

¹H NMR (400 MHz, CDCl₃-d) δ = 10.71 (s, 1H), 8.63 (s, 1H), 8.13 (d, J = 2.0 Hz, 1H), 7.69 (d, J = 8.6 Hz, 1H), 7.58 (dd, J = 1.8, 8.6 Hz, 1H), 6.81 (t, J = 73.0 Hz, 1H), 5.86 (s, 1H), 4.02 (s, 3H), 2.25 (s, 3H).

### Step 7: 4-[(3-Amino-5-bromo-6-methoxy-2-pyridyl)amino]-2-[3-(difluoromethoxy)-5-methyl-pyrazol-1-yl]benzonitrile

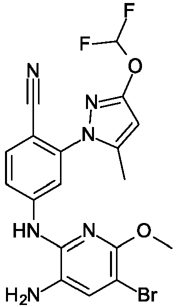

To a yellow solution of 4-[(5-bromo-6-methoxy-3-nitro-2-pyridyl)amino]-2-[3-(difluoromethoxy)-5-methyl-pyrazol-1-yl]benzonitrile (1.5 g, 3.03 mmol, 1 equiv.) in ethanol (25 mL) were added iron (845.7 mg, 15.1 mmol, 5 equiv.), NH₄Cl (1.62 g, 30.3 mmol, 10 equiv.) and water (12.5 mL). The yellow suspension was stirred at 50 °C for 16 hours under a nitrogen atmosphere. Then, MeOH (50 mL) was added to the mixture and stirring was continued at 50 °C for 2 hours. The mixture was filtered while hot. The filtrate was concentrated. The crude product was dissolved in ethyl acetate and washed with H₂O. The combined organics were washed with brine, dried over Na₂SO₄, filtered and concentrated. The crude product was purified by silica gel chromatography using a ethyl acetate/petroleum ether gradient as eluent to give the title compound (0.917 g, 2.36 mmol, 65% yield) as brown solid. LC-MS: m/z = 465.0/467.0 [M+H]⁺, ESI pos.

¹H NMR (400 MHz, CDCl₃-d) δ = 7.89 (s, 1H), 7.68 - 7.62 (m, 1H), 7.61 - 7.55 (m, 1H), 7.46 - 7.37 (m, 2H), 6.93 (t, J = 73.2 Hz, 1H), 5.92 (s, 1H), 3.94 (s, 3H), 2.32 (s, 3H).

### Step 8: 4-(6-Bromo-5-methoxy-imidazo[4,5-b]pyridin-3-yl)-2-[3-(difluoromethoxy)-5-methyl-pyrazol-1-yl]benzonitrile

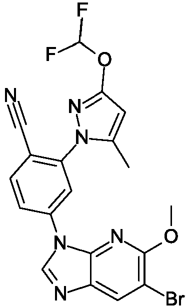

To a yellow solution of 4-[(3-amino-5-bromo-6-methoxy-2-pyridyl)amino]-2-[3-(difluoromethoxy)-5-methyl-pyrazol-1-yl]benzonitrile (1.1 g, 2.36 mmol, 1 equiv.) and trimethyl orthoformate (2.51 g, 23.64 mmol, 10 equiv.) in ethanol (40 mL) was added TsOH (40.7 mg, 0.240 mmol, 0.1 equiv.). The resulting suspension was stirred at 80 °C for 2 hours. The solid was dissolving slowly during this process. The reaction mixture was poured into water and extracted with ethyl acetate. The combined organic layers were dried over Na₂SO₄, filtered and concentrated. The crude product was purified by silica gel chromatography using a petroleum ether/ethyl acetate gradient as eluent to provide the title compound (850 mg, 1.79 mmol, 76% yield) as brown solid. LC-MS: m/z = 475.0/477.0[M+H]⁺, ESI pos.

¹H NMR (400 MHz, CDCl₃-d) δ = 8.35 (s, 1H), 8.32 (s, 1H), 8.22 (d, J = 2.0 Hz, 1H), 8.12 - 8.07 (m, 1H), 8.03 - 7.98 (m, 1H), 6.96 (t, J = 72.9 Hz, 1H), 6.01 (s, 1H), 4.09 (s, 3H), 2.41 (s, 3H).

### Step 9: Ethyl 3-(benzhydrylideneamino)-6-methyl-pyridazine-4-carboxylate

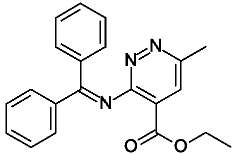

To a solution of ethyl 3-chloro-6-methyl-pyridazine-4-carboxylate (2.0 g, 9.97 mmol, 1 equiv.) and alpha-phenyl benzenemethanimine (2.5 mL, 14.9 mmol, 1.5 equiv.) in 1,4-dioxane (25 mL) were added Cs₂CO₃ (9.74 g, 29.9 mmol, 3 equiv.) and Xantphos Pd G4 (0.96 g, 1 mmol, 0.1 equiv.). The mixture was stirred at 100 °C for 16 hours under an nitrogen atmosphere. The reaction mixture was poured into water and extracted with EtOAc. The organics were dried over sodium sulfate, filtered and concentracted. The crude product was purified by silica gel chromatography to give the titel compound (1.4 g, 4.1 mmol, 41% yield) as yellow solid. The water phase was lyophilized. The residue was treated with ethyl acetate/methanol 10:1 and stirred for 30 minutes. The mixture was filtered, and the mother liquid was concentrated to give another crop of title compound (800 mg, 2.52 mmol, 25% yield) as yellow solid. LC-MS: 346.1 [M+H]⁺, ESI pos.

¹H NMR (400 MHz, CDCl₃-d) δ = 7.92 - 7.19 (m, 11H), 4.31 (q, J = 7.1 Hz, 2H), 2.68 (s, 3H), 1.27 (t, J = 7.2 Hz, 3H).

### Step 10: 3-(Benzhydrylideneamino)-6-methyl-pyridazine-4-carboxylic acid

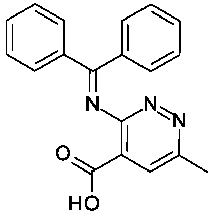

To a mixture of ethyl 3-(benzhydrylideneamino)-6-methyl-pyridazine-4-carboxylate (1.4 g, 4.05 mmol, 1 equiv.) in THF (5 mL), MeOH (5 mL), water (2.5 mL) was added LiOH (242.7 mg, 10. mmol, 2.5 equiv.). The mixture was stirred at 25 °C for 1 hour, then concentrated under reduced pressure to give the title compound (1.2 g, 3.8 mmol, 93% yield) as yellow solid. The crude product was used directly in the next step without further purification. LC-MS: 318.0 [M+H]⁺, ESI pos.

### Step 11: 3-(Benzhydrylideneamino)-N,N, 6-trimethyl-pyridazine-4-carboxamide

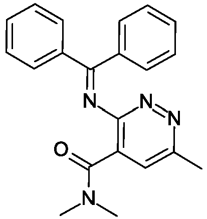

To a solution of 3-(benzhydrylideneamino)-6-methyl-pyridazine-4-carboxylic acid (800.0 mg, 2.52 mmol, 1 equiv.) in DMF (12 mL) were added dimethylamine hydrochloride (411.1 mg, 5.04 mmol, 2 equiv.), DIPEA (1.3 mL, 7.56 mmol, 3 equiv.) and HATU (1.78 g, 7.56 mmol, 3 equiv.). The reaction mixture was stirred at 30 °C for 16 hours. The mixture was used in the next step directly. LC-MS: 345.1 [M+H]⁺, ESI pos.

### Step 12: 3-Amino-N,N,6-trimethyl-pyridazine-4-carboxamide

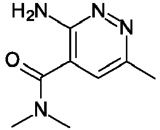

A solution of HCl in 1,4-dioxane (10.0 mL, 40 mmol, 6.9 equiv.) was added to the mixture of 3-(benzhydrylideneamino)-N,N,6-trimethyl-pyridazine-4-carboxamide (2.0 g, 5.81 mmol, 1 equiv.) at 0 °C, and stirred at 30 °C for 1 hour. The mixture was purified by prep-HPLC using 0.05% aqueous ammonia hydroxide/acetonitrile gradient as eluent, then by a second prep-HPLC using a 10 mmol ammonium carbonate/acetonitrile gradient as eluent to give the title compound (500 mg, 2.77 mmol, 45% yield) as white solid after lyophilization. LC-MS: 181.0 [M+H]⁺, ESI pos.

¹H NMR (400 MHz, MeOH-d₄) δ = 7.28 (s, 1H), 3.12 (s, 3H), 2.99 (s, 3H), 2.52 (s, 3H).

### Step 13: 3-[[3-[4-Cyano-3-[3-(difluoromethoxy)-5-methyl-pyrazol-1-yl]phenyl]-5-methoxy-imidazo[4,5-b]pyridin-6-yl]amino]-N,N,6-trimethyl-pyridazine-4-carboxamide

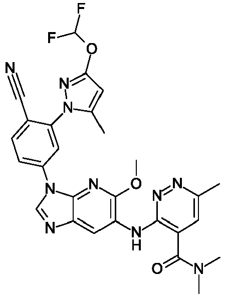

To a colorless solution of 4-(6-bromo-5-methoxy-imidazo[4,5-b]pyridin-3-yl)-2-[3-(difluoromethoxy)-5-methyl-pyrazol-1-yl]benzonitrile (30.0 mg, 0.06 mmol, 1 equiv.) and 3-amino-N,N,6-trimethyl-pyridazine-4-carboxamide (3 mg, 0.13 mmol, 2 equiv.) in 1,4-dioxane (2 mL) were added [t-BuBrettPhos Pd(allyl)]OTf (10 mg, 0.01 mmol, 0.2 equiv.) and Cs₂CO₃ (61.7 mg, 0.19 mmol, 3 equiv.). The reaction mixture was flushed with nitrogen, stirred at 80 °C for 2 hours, then cooled to RT, poured into water and extracted with ethyl acetate. The combined organic layers were dried over Na₂SO₄, filtered and concentrated. The crude product was purified by prep-HPLC using 0.1% formic acid/acetonitrile gradient as eluent followed by prep-TLC using DCM/MeOH as eluent to give the title compound (5.1 mg, 0.01 mmol, 14% yield) as white solid by lyophilization. LC-MS: m/z = 575.2 [M+H]⁺, ESI pos.

¹H NMR (400 MHz, MeOH-d₄) δ = 9.08 (s, 1H), 8.76 (s, 1H), 8.53 (s, 1H), 8.46 (br d, J = 9.5 Hz, 1H), 8.16 (d, J = 8.4 Hz, 1H), 7.49 (s, 1H), 7.30 - 6.89 (m, 1H), 6.12 (s, 1H), 4.13 (s, 3H), 3.19 (br s, 3H), 3.11 (br s, 3H), 2.63 (s, 3H), 2.41 (s, 3H).

### Example 5

### 1-[2-(1-Hydroxyethyl)-5-[6-(6-methylpyridazin-3-yl)oxypyrazolo[1,5-a]pyrimidin-3-yl]phenyl]-5-methylpyrazole-3-carbonitrile

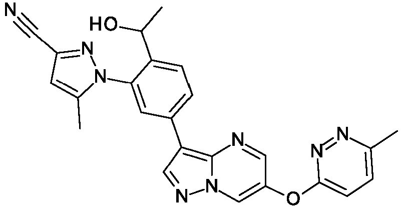

### Step 1: 6-(6-Methylpyridazin-3-yl)oxypyrazolo[1,5-a]pyrimidine

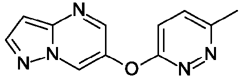

A solution of pyrazolo[1,5-a]pyrimidin-6-ol (CAS 1580489-59-3; 1 g, 7.4 mmol, 1.0 equiv.) was dissolved in DMF (50 mL) and treated with 3-chloro-6-methyl-pyridazine (1.14 g, 8.88 mmol, 1.2 equiv.) and Cs₂CO₃ (4.82 g, 14.8 mmol, 2.0 equiv.) at RT. The mixture was stirred for 16 hours at 100 °C, then cooled to RT, diluted with water and extracted with ethyl acetate. The organic layers were washed with brine, dried over MgSO₄ and concentrated. The crude product was purified by silica gel chromatography using a DCM/MeOH gradient as eluent to obtain the title compound (259 mg, 12% yield) as light yellow solid. LC-MS: m/z = 228.6 [M+H]⁺, ESI pos.

### Step 2: 3-Bromo-6-(6-methylpyridazin-3-yl)oxy-pyrazolo[1,5-a]pyrimidine

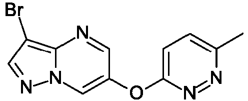

To a solution of 6-(6-methylpyridazin-3-yl)oxypyrazolo[1,5-a]pyrimidine (250 mg, 1.1 mmol, 1.0 equiv.) in acetic acid (10 mL), was added dropwise Br₂ (246 mg, 79 uL, 1.54 mmol, 1.4 equiv.). This mixture was stirred for 1 hour at RT. More Br₂ (246 mg, 79 uL, 1.54 mmol, 1.4 equiv.) was added, then stirred 1 hour at RT. The mixture was pourred onto water and extracted with DCM. The organic layers were dried over Na₂SO₄, filtered and concentrated. The crude product was purrified by silica gel chromatography using a DCM/MeOH gradient as eluent to give the title compound (121 mg, 34% yield) as light brown liquid. LC-MS: m/z = 307.9 [M+H]⁺, ESI pos.

### Step 3: 1-[2-Acetyl-5-[6-(6-methylpyridazin-3-yl)oxypyrazolo[1,5-a]pyrimidin-3-yl]phenyl]-5-methyl-pyrazole-3-carbonitrile

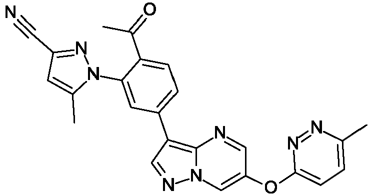

A solution of 1-[2-acetyl-5-(4,4,5,5-tetramethyl-1,3,2-dioxaborolan-2-yl)phenyl]-5-methylpyrazole-3-carbonitrile (example 2, step 2; 60 mg, 0.17 mmol, 1.0 equiv.) in 1,4-dioxane (2 mL) was treated with 3-bromo-6-(6-methylpyridazin-3-yl)oxy-pyrazolo[1,5-a]pyrimidine (52.3 mg, 0.17 mmol, 1.0 equiv.), K₂CO₃ (59 mg, 0.43 mmol, 2.5 equiv.) in water (0.5 mL) and then with Pd(dppf)Cl₂·CH₂Cl₂ complex (28 mg, 0.034 mmol, 0.2 equiv.). The reaction mixture was stirred at 50 °C for 90 minutes, then poured into water and extracted with ethyl acetate. The combined organic layers were dried over MgSO₄, filtered and concetranted. The crude product was purified by silica gel chromatography using a heptane/ethyl acetate gradient as eluent to obtain the title compound (35 mg, 43% yield) as yellow solid. LC-MS: m/z = 451.2 [M+H]⁺, ESI pos.

### Step 4: 1-[2-(1-Hydroxyethyl)-5-[6-(6-methylpyridazin-3-yl)oxypyrazolo[1,5-a]pyrimidin-3-yl]phenyl]-5-methylpyrazole-3-carbonitrile

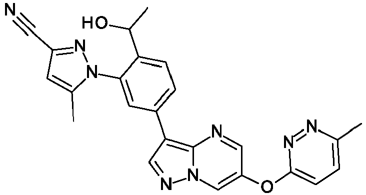

A suspension of 1-[2-acetyl-5-[6-(6-methylpyridazin-3-yl)oxypyrazolo[1,5-a]pyrimidin-3-yl]phenyl]-5-methyl-pyrazole-3-carbonitrile (35 mg, 0.08 mmol, 1.0 equiv.) in isopropanol (2.3 mL) and THF (2.3 mL) was treated with NaBH₄ (5.9 mg, 0.16 mmol, 2.0 equiv.). The mixture was stirred for 2 hours at RT, then diluted with water and extracted with ethyl acetate. The organic layers were dried over MgSO₄ and concentrated. The crude product was purified by silica gel chromatography using a heptane/ethyl acetate gradient as eluent to obtain the title compound (14 mg, 40% yield) as yellow powder. LC-MS: m/z = 457.2 [M+H]⁺, ESI+

### Example 6

### 3-[[3-[4-Cyano-3-[3-(difluoromethoxy)-5-methyl-pyrazol-1-yl]phenyl]imidazo[4,5-b]pyridin-6-yl]amino]-N,N,6-trimethyl-pyridazine-4-carboxamide

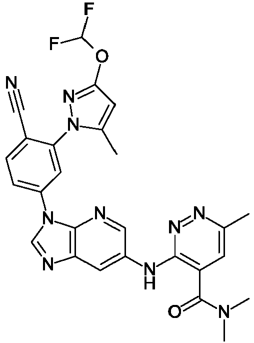

### Step 1: 2-[3-(Difluoromethoxy)-5-methyl-pyrazol-1-yl]-4-[(3,5-dinitro-2-pyridyl)amino]benzonitrile

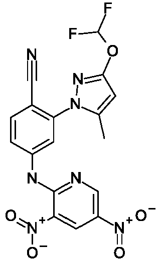

To a yellow solution of 4-amino-2-[3-(difluoromethoxy)-5-methyl-pyrazol-1-yl]benzonitrile (example 4, step 5; 400 mg, 1.51 mmol, 1.0 equiv.) in methanol (30 mL) was added 2-chloro-3,5-dinitropyridine (CAS 2578-45-2; 308 mg, 1.51 mmol, 1.0 equiv.) and stirred at 70 °C for 2 hours. More 2-chloro-3,5-dinitropyridine (154 mg, 0.76 mmol, 0.5 equiv.) was added to the mixture and stirring was continued at 70 °C for 16 hours. The mixture was concentrated. The crude product was purified by silica gel chromatography using a petroleum ether/ethyl acetate gradient as eluent to give the title compound (530.0 mg, 1.23 mmol, 81% yield) as yellow solid. LC-MS: m/z = 432.1 [M+H]⁺, ESI pos.

¹H NMR (400 MHz, CDCl₃-d) δ = 10.82 (s, 1H), 9.42 (s, 2H), 8.12 (d, J = 1.1 Hz, 1H), 7.92 - 7.79 (m, 2H), 6.97 (t, J = 72.9 Hz, 1H), 6.00 (s, 1H), 2.39 (s, 3H).

### Step 2: 4-[(3,5-Diamino-2-pyridyl)amino]-2-[3-(difluoromethoxy)-5-methyl-pyrazol-1-yl]benzonitrile

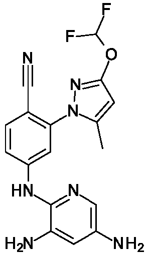

To a yellow solution of 2-[3-(difluoromethoxy)-5-methyl-pyrazol-1-yl]-4-[(3,5-dinitro-2-pyridyl)amino]benzonitrile (530.0 mg, 1.23 mmol, 1.0 equiv.) in EtOH (20 mL) were added Fe (686 mg, 12.3 mmol, 10.0 equiv.), NH₄Cl (1.3 g, 24.6 mmol, 20.0 eq) and water (10 mL). The yellow suspension was stirred at 50 °C for 2 hours under a nitrogen atmosphere. Then, MeOH (50 mL) was added to the mixture and stirring was continued at 50 °C for 30 minutes. The mixture was filtered while hot. The filtrate was concentrated. The residue was dissolved in ethyl acetate and washed with saturated NaHCO₃ solution. The combined organic layers were washed with brine, dried over Na₂SO₄, filtered and concentrated. The crude product was purified by silica column using an ethyl acetate/MeOH gradient as eluent to provide the title compound (330.0 mg, 0.89 mmol, 72% yield) as dark green solid. LC-MS: m/z = 372.0 [M+H]⁺, ESI pos.

¹H NMR (400 MHz, CDCl₃-d) δ = 7.54 (d, J = 9.2 Hz, 1H), 7.41 (d, J = 2.4 Hz, 1H), 7.11 (s, 1H), 6.93 - 6.88 (m, 2H), 6.74 (s, 1H), 6.48 (d, J = 2.6 Hz, 1H), 6.20 (s, 1H), 5.89 (s, 1H), 3.51 (s, 4H), 2.28 (s, 3H).

### Step 3: 4-(6-Aminoimidazo[4,5-b]pyridin-3-yl)-2-[3-(difluoromethoxy)-5-methyl-pyrazol-1-yl]benzonitrile

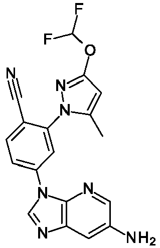

To a dark green solution of 4-[(3,5-diamino-2-pyridyl)amino]-2-[3-(difluoromethoxy)-5-methyl-pyrazol-1-yl]benzonitrile (250.0 mg, 0.67 mmol, 1.0 equiv.) in methanol (250 mL) were added trimethyl orthoformate (714 mg, 6.7 mmol, 10.0 equiv.) and TsOH (11.6 mg, 0.07 mmol, 0.1 equiv.). The green suspension was stirred at 80 °C for 60 hours. The mixture was concentrated to remove most of methanol. Then, the mixture was poured into NaHCO₃ and extracted with ethyl acetate. The combined organics were dried over Na₂SO₄, filtered and concentrated. The crude product was purified by prep-HPLC using 0.1% formic acid/acetonitrile gradient as eluent to give the title compound (35.0 mg, 0.09 mmol, 14% yield) as white solid. LC-MS: m/z = 382.1 [M+H]⁺, ESI pos.

### Step 4: 4-(6-Bromoimidazo[4,5-b]pyridin-3-yl)-2-[3-(difluoromethoxy)-5-methyl-pyrazol-1-yl]benzonitrile

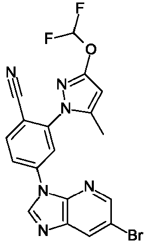

To a dark green suspension of copper(I) bromide (11.3 mg, 0.08 mmol, 1.0 equiv.) in acetonitrile (6 mL) was added isoamyl nitrite (18.4 mg, 0.16 mmol, 2.0 equiv.) and stirred at 65 °C for 15 minutes under an argon atmosphere. The reaction mixture was cooled to room temperature. More 4-(6-aminoimidazo[4,5-b]pyridin-3-yl)-2-[3-(difluoromethoxy)-5-methyl-pyrazol-1-yl]benzonitrile (30.0 mg, 0.08 mmol, 1.0 equiv.) in acetonitrile (1 mL) was added to the reaction mixture which was stirred at 65 °C for 30 minutes, then poured into water. The aqueous solution was extracted with ethyl acetate. The combined organics were washed with brine, dried over Na₂SO₄ and concentrated. The crude product was purified by prep-TLC using ethyl acetate as eluent to provide the title compound (30.0 mg, 0.07 mmol, 86% yield) as white solid. LC-MS: m/z = 445.0/447.0 [M+H]⁺, ESI pos.

### Step 5: 3-[[3-[4-Cyano-3-[3-(difluoromethoxy)-5-methyl-pyrazol-1-yl]phenyl]imidazo[4,5-b]pyridin-6-yl]amino]-N,N,6-trimethyl-pyridazine-4-carboxamide

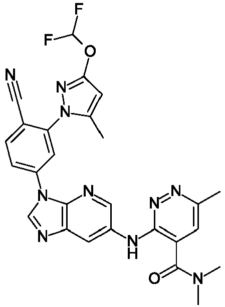

To a yellow solution of 3-amino-*N,N*,6-trimethyl-pyridazine-4-carboxamide (example 4, step 12; 24.3 mg, 0.13 mmol, 2.0 equiv.) in 1,4-dioxane (5 mL) were added 4-(6-bromoimidazo[4,5-b]pyridin-3-yl)-2-[3-(difluoromethoxy)-5-methyl-pyrazol-1-yl]benzonitrile (30.0 mg, 0.07 mmol, 1.0 equiv.), [t-BuBrettPhos Pd(allyl)]OTf (10.53 mg, 0.01 mmol, 0.2 equiv.) and Cs₂CO₃ (65.9 mg, 0.2 mmol, 3.0 equiv.). The reaction mixture was flushed with nitrogen. The yellow suspension was stirred at 80 °C for 1 hour, then poured into water and extracted with EtOAc. The combined organics were washed with brine, dried over Na₂SO₄, filtered and concentrated. The crude product was purified by prep-TLC using dichloromethane/methanol 10:1, then by prep-HPLC using 0.1% formic acid/acetonitrile gradient as eluent to give the title compound (4.7 mg, 0.01 mmol, 13% yield) as white lyophilisate. LC-MS: m/z = 545.3 [M+H]⁺, ESI pos.

¹H NMR (400 MHz, MeOH-d₄) δ = 8.97 (s, 1H), 8.70 (d, J = 2.3 Hz, 1H), 8.57 (dd, J = 2.2, 3.7 Hz, 2H), 8.44 (dd, J = 2.2, 8.6 Hz, 1H), 8.17 (d, J = 8.6 Hz, 1H), 7.43 (s, 1H), 7.10 (t, J = 73.2 Hz, 1H), 6.13 (s, 1H), 3.16 (s, 3H), 3.08 (s, 3H), 2.61 (s, 3H), 2.43 (s, 3H).

### Example 7

### 1-[3-Fluoro-2-(1-hydroxyethyl)-5-[6-[(6-methylpyridazin-3-yl)amino]imidazo[4,5-b]pyridin-3-yl]phenyl]-5-methyl-pyrazole-3-carbonitrile

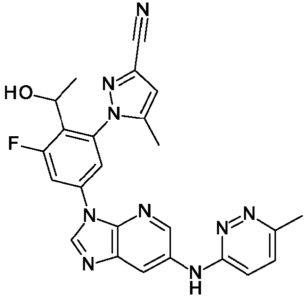

### Step 1: 2-(1-Ethoxyvinyl)-1,3-difluoro-5-nitro-benzene

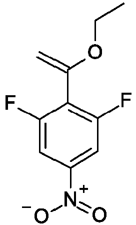

To a solution of 4-bromo-3,5-difluoronitrobenzene (CAS 167415-27-2; 5.0 g, 21.01 mmol, 1.0 equiv.) in toluene (100 mL) was added bis(triphenylphosphine)palladium(II)chloride (1.47 g, 2.1 mmol, 0.1 equiv.) and tributyl(1-ethoxyvinyl)tin (11.38 g, 31.51 mmol, 1.5 equiv.). The mixture was flushed with nitrogen and stirred at 110 °C for 14 hours. The reaction mixture was cooled to room temperature, quenched with saturated potassium fluoride and stirred at room temperature for 12 hours. The aqueous phase was extracted with ethyl acetate. The organics were washed with brine and dried over sodium sulfate, filtered and concentracted. The crude product was purified by flash chromatography using a petroleum ether/ethyl acetate gradient as eluent to provide the title compound (5.0 g, 21.8 mmol, 83% yield) as orange liquid.

¹H NMR (400 MHz, CDCl₃-d) δ = 7.86 - 7.78 (m, 2H), 4.68 (d, J = 3.1 Hz, 1H), 4.45 (d, J = 2.9 Hz, 1H), 3.97 (q, J = 7.0 Hz, 2H), 1.43 - 1.39 (m, 3H).

### Step 2: 1-(2,6-Difluoro-4-nitro-phenyl)ethanone

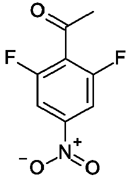

To a orange solution of 2-(1-ethoxyvinyl)-1,3-difluoro-5-nitro-benzene (5.0 g, 21.8 mmol, 1.0 equiv.) in THF (10 mL) was added HCl (10.0 mL, 10.0 mmol, 0.46 equiv.). The mixture was heated to 30 °C and stirred for 2 days, then basified by saturated aqueous NaHCO₃ to pH 8. The resulting mixture was extracted by ethyl acetate. The organic layer was dried over anhydrous sodium sulfate and concentrated. The crude product was purified by silica gel chromatography using a petroleum ether/ethyl acetate gradient as eluent to provide the title compound (2.8 g, 13.9 mmol, 61% yield) as orange liquid.

¹H NMR (400 MHz, CDCl₃-d) δ = 7.91 - 7.85 (m, 2H), 2.67 (t, J = 1.3 Hz, 3H).

### Step 3: 1-(2-Acetyl-3-fluoro-5-nitro-phenyl)-5-methyl-pyrazole-3-carbonitrile

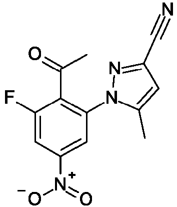

To a yellow solution of 1-(2,6-difluoro-4-nitro-phenyl)ethanone (2.8 g, 13.92 mmol, 1.0 equiv.), 5-methyl-1H-pyrazole-3-carbonitrile (1.19 g, 11.14 mmol, 0.8 equiv.) in DMSO (20 mL) was added DIPEA (5.4 g, 41.8 mmol, 3.0 equiv.). The mixture was stirred at 100 °C for 12 hours to give a brown solution, then quenched with water and extracted by ethyl acetate. The combined organics was washed by brine, dried over anhydrous sodium sulfate and concentrated. The crude product was purified by silica gel chromatography using a petroleum ether/ethyl acetate gradient as eluent to give the title compound (1.3 g, 4.51 mmol, 29% yield; contains 30% of the regiosimer 2-(2-acetyl-3-fluoro-5-nitro-phenyl)-5-methyl-pyrazole-3-carbonitrile) as white solid. LC-MS: m/z = 289.1 [M+H]⁺, ESI pos.

### Step 4: 1-(2-Acetyl-5-amino-3-fluoro-phenyl)-5-methyl-pyrazole-3-carbonitrile

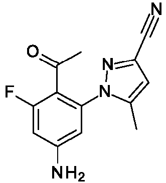

To a colorless solution of 1-(2-acetyl-3-fluoro-5-nitro-phenyl)-5-methyl-pyrazole-3-carbonitrile (1.3 g, 4.51 mmol, 1.0 equiv.; contains 30% of regioisomer) in EtOH (14 mL) were added NH₄Cl (2.41 g, 45.1 mmol, 10.0 equiv.), water (7 mL) and Fe (1.26 mg, 22.6 mmol, 5.0 equiv.). Then the mixture was heated to 50 °C and stirred at 50 °C for 2 hours to give a black suspension. The reaction mixture was filtered through a pad of celite at 50 °C, the filter cake was washed by methanol (30 mL x 5), the filtrate was concentrated under vacuo. To the resulting mixture was added water (50 mL) and the product was extracted with ethyl acetate (20 mL x 3). The organic phase was washed with brine (20 mL x 3), dried over anhydrous sodium sulfate, filtered, concentrated under reduced pressure to give the title compound 850.0 mg, 3.29 mmol, 66% yield; contains 30 % of regioisomer 2-(2-acetyl-5-amino-3-fluoro-phenyl)-5-methyl-pyrazole-3-carbonitrile) as grey solid. LC-MS: 259.1 [M+H]⁺, ESI pos.

### Step 5: 1-[2-Acetyl-5-[(3,5-dinitro-2-pyridyl)amino]-3-fluoro-phenyl]-5-methyl-pyrazole-3-carbonitrile

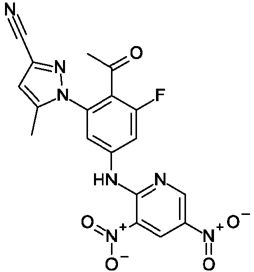

To a brown solution of 2-chloro-3,5-dinitropyridine (CAS 2578-45-2; 693.57 mg, 3.41 mmol, 1.0 equiv.), 1-(2-acetyl-5-amino-3-fluoro-phenyl)-5-methyl-pyrazole-3-carbonitrile (880.0 mg, 3.41 mmol, 1.0 equiv.; contains 30% of regioisomer) in MeOH (10 mL) was heated to 70 °C and stirred at 70 °C for overnight to give a brown suspension, then concentrated. The residue was taken up in water and extracted by ethyl acetate. The organic phase was dried over anhydrous sodium sulfate, filtered and concentrated. The crude product was purified by flash chromatography using a petroleum/ethylacetate gradient as eluent to give the title compound (1.3 g, 3.1 mmol, 83% yield; contains 30% regioismer 2-[2-acetyl-5-[(3,5-dinitro-2-pyridyl)amino]-3-fluoro-phenyl]-5-methyl-pyrazole-3-carbonitrile) as orange solid. LC-MS: 426.1 [M+H]⁺, ESI pos.

### Step 6: 1-[2-Acetyl-3-fluoro-5-[6-[(6-methylpyridazin-3-yl)amino]imidazo[4,5-b]pyridin-3-yl]phenyl]-5-methyl-pyrazole-3-carbonitrile

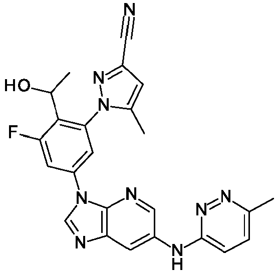

In analogy to example 6 (steps 7 - 10), and starting from 1-[2-acetyl-5-[(3,5-dinitro-2-pyridyl)amino]-3-fluoro-phenyl]-5-methyl-pyrazole-3-carbonitrile (containing 30% of regioisomer), the title compound was obtained as brown solid. The desired regioisomer was separated from the undesired compound after the last step by crystallization from ethyl acetate/water during workup of the reaction mixture. LC-MS: 468.2 [M+H]⁺, ESI pos.

¹H NMR (400 MHz, DMSO-d₆) δ = 9.53 (br s, 1H), 9.10 (s, 1H), 8.89 (d, J = 1.9 Hz, 1H), 8.65 (d, J = 1.5 Hz, 1H), 8.55 (br d, J = 11.4 Hz, 1H), 8.37 (s, 1H), 7.40 (d, J = 9.1 Hz, 1H), 7.14 (br d, J = 9.0 Hz, 1H), 7.07 (s, 1H), 3.58 (s, 3H), 2.41 (s, 3H), 2.32 (s, 3H).

### Step 7: 1-[3-Fluoro-2-(1-hydroxyethyl)-5-[6-[(6-methylpyridazin-3-yl)amino]imidazo[4,5-b]pyridin-3-yl]phenyl]-5-methyl-pyrazole-3-carbonitrile

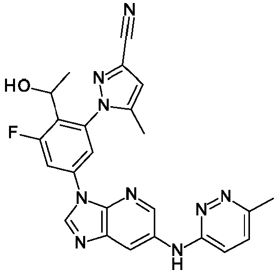

To a brown suspension of 1-[2-acetyl-3-fluoro-5-[6-[(6-methylpyridazin-3-yl)amino]imidazo[4,5-b]pyridin-3-yl]phenyl]-5-methyl-pyrazole-3-carbonitrile (80.0 mg, 0.17 mmol, 1.0 equiv.) in DMSO (0.5 mL) and methanol (1 mL) was added NaBH₄ (32.37 mg, 0.86 mmol, 5.0 equiv.) at 20 °C. The reaction mixture was heated to 30 °C and stirred for 1 hour. It was treated again with NaBH₄ (32.4 mg, 0.86 mmol, 5.0 equiv.) and stirred at 30 °C for 1 hour. The reaction mixture was treated with saturated aqueous NH₄Cl (1 mL), extracted with ethyl acetate. The combined organic layers were dried over Na₂SO₄, filtered and concentrated. The crude residue was purified by prep-HPLC using 0.225% aqueous formic acid/acetonitrile gradient as eluent to give the title compound (6.4 mg, 0.01 mmol, 7.9% yield) as white solid. LC-MS: 470.2 [M+H]⁺, ESI pos

¹H NMR (400 MHz, DMSO-d₆) δ = 9.46 (s, 1H), 9.01 (s, 1H), 8.89 - 8.86 (m, 1H), 8.60 (t, J = 1.8 Hz, 1H), 8.36 (dd, J = 2.1, 12.2 Hz, 1H), 8.05 (s, 1H), 7.39 (d, J = 9.2 Hz, 1H), 7.12 (d, J = 9.0 Hz, 1H), 7.04 (s, 1H), 5.37 (br s, 1H), 4.61 - 4.00 (m, 1H), 2.53 (d, J = 2.0 Hz, 3H), 2.27 (s, 3H), 1.43 (br d, J = 6.5 Hz, 3H).

### Example 8

### 1-[2-[3-(Difluoromethyl)-5-methyl-pyrazol-1-yl]-4-[6-[(6-methylpyridazin-3-yl)amino]pyrazolo[1,5-a]pyrimidin-3-yl]phenyl]ethanol

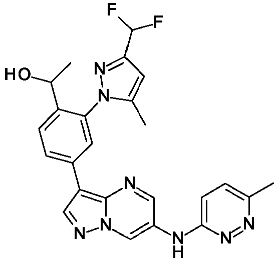

### Step 1: 4-Bromo-2-[3-(difluoromethyl)-5-methyl-pyrazol-1-yl] benzonitrile

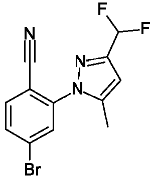

To a colorless solution of 4-bromo-2-fluorobenzonitrile (CAS 105942-08-3; 3.0 g, 15.0 mmol, 1.0 equiv.) in DMF (60 mL) was added 3-(difluoromethyl)-5-methyl-1H-pyrazole (1.98 g, 15.0 mmol, 1.0 equiv.) and potassium carbonate (6.22 g, 45.0 mmol, 3.0 equiv.). The white suspension was stirred at 20 °C for 16 hours. The mixture was poured into water and extracted with ethyl acetate. The combined organics were washed with brine, dried over Na₂SO₄, filtered and concentrated. The crude product was purified by silica gel chromatography using a petroleum ether/ethyl acetate gradient (4.0 g, 12.8 mmol, 85% yield) as white solid. LC-MS: 312.0/314.0 [M+H]⁺, ESI pos.

¹H NMR (400 MHz, CDCl₃-d) δ = 7.80 - 7.68 (m, 3H), 6.72 (t, J = 54.9 Hz, 1H), 6.52 (s, 1H), 2.37 (s, 3H).

### Step 2: 4-Bromo-2-[3-(difluoromethyl)-5-methyl-pyrazol-1-yl] benzaldehyde

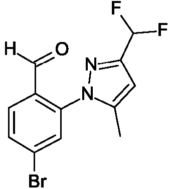

To a colorless solution of 4-bromo-2-[3-(difluoromethyl)-5-methyl-pyrazol-1-yl]benzonitrile (3) (3.0 g, 9.6 mmol, 1.0 equiv.) in THF (45 mL) was added dropwise a 1M DIBAL-H solution in toluene (24.0 mL, 24.0 mmol, 2.5 equiv.) at 0 °C under a nitrogen atmosphere. The yellow solution was stirred at 0 °C for 2 hours. The reaction mixture was quenched with 1N HCl and extracted with ethyl acetate. The combined organic layers were dried over Na₂SO₄, filtered and concentrated. The crude product was purified by silica gel chromatography using a petroleum ether/ethyl acetate gradient as eluent (1.2 g, 3.8 mmol, 40% yield) as colorless oil. LC-MS: 314.8/316.8 [M+H]⁺, ESI pos.

¹H NMR (400 MHz, CDCl₃-d) δ = 9.58 (d, J = 0.6 Hz, 1H), 7.96 (d, J = 8.3 Hz, 1H), 7.86 - 7.73 (m, 1H), 7.64 (d, J = 1.8 Hz, 1H), 6.70 (t, J = 54.8 Hz, 1H), 6.54 (s, 1H), 2.31 (s, 3H)

### Step 3: 2-[3-(Difluoromethyl)-5-methyl-pyrazol-1-yl]-4-(4,4,5,5-tetramethyl-1,3,2-dioxaborolan-2-yl)benzaldehyde

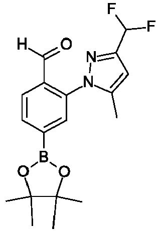

To a colorless solution of 4-bromo-2-[3-(difluoromethyl)-5-methyl-pyrazol-1-yl]benzaldehyde (1.60 g, 5.1 mmol, 1.0 equiv.) in 1,4-dioxane (40 mL) were added bis(pinacolato)diboron (2.58 g, 10.2 mmol, 2.0 equiv.), 1,1'-bis(diphenylphosphino)ferrocenepalladium(II) dichloride, toluene complex (417 mg, 0.51 mmol, 0.1 equiv.) and potassium acetate (0.95 mL, 15.23 mmol, 3.0 equiv.). The reation mixture was flushed with nitrogen and stirred at 100 °C for 2 hours, then poured into water and extracted with ethyl acetate. The combined organic layers were washed with brine, dried over Na₂SO₄, filtered and concentrated. The crude product was purified by silica gel chromatography using a petroleum ether/ethyl acetate gradient as eluent to provide the title compound (2.4 g, 6.63 mmol, 91% yield) as yellow oil. LC-MS: 281.1 [M+H]⁺, ESI pos.

¹H NMR (400 MHz, CDCl₃-d) δ = 9.69 - 9.60 (m, 1H), 8.06 (s, 2H), 7.85 (s, 1H), 6.85 - 6.55 (m, 1H), 6.51 (s, 1H), 2.26 (s, 3H), 1.40 - 1.35 (m, 12H)

### Step 4: N-(6-Methylpyridazin-3-yl)pyrazolo[1,5-a]pyrimidin-6-amine

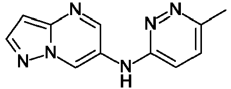

To a yellow solution of 3-amino-6-methylpyridazine (1.32 g, 12.1 mmol, 1.2 equiv.) in 1,4-dioxane (20 mL) was added 6-bromopyrazolo[1,5-a]pyrimidine (2.0 g, 10.1 mmol, 1.0 equiv.) and cesium carbonate (9.9 g, 30.3 mmol, 3.0 equiv.). The air of the flask was replaced by nitrogen for three times. Then, [t-BuBrettPhos Pd(allyl)]OTf (1.58 g, 2.02 mmol, 0.2 equiv.) was added to the mixture which was then stirred at 80 °C for 2 hours. The mixture was poured into water, extracted with EtOAc and filtered to obtain the insoluble crude product as brown solid. This solid was triturated with ethyl acetate/methanol 20/1 (30 mL) at 25 °C for 30 minutes to give the title compound (1.3 g, 5.75 mmol, 57% yield) as a brown solid. LC-MS = 277.0 [M+H]⁺, ESI pos.

### Step 5: 3-Iodo-N-(6-methylpyridazin-3-yl)pyrazolo[1,5-a]pyrimidin-6-amine

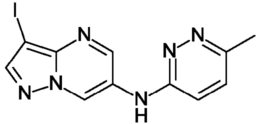

To a yellow solution of N-(6-methylpyridazin-3-yl)pyrazolo[1,5-a]pyrimidin-6-amine (1.3 g, 5.75 mmol, 1.0 equiv.) in DMF (15 mL) was added NIS (1.29 g, 11.49 mmol, 2.0 equiv.). The reaction was stirred at 30 °C for 16 hours. The reaction mixture was again treated with NIS (325 mg, 2.9 mmol, 0.5 equiv.) and stirring was continued at 30 °C for another 4 hours. The reaction mixture was concentrated. The crude product was triturated with 10 mL ethyl acetate at room temperature for 30 minutes. The mixture was filtered and the filter cake was concentrated to give the title compound (1.3 g, 3.7 mmol, 64% yield) as a brown solid. LC-MS = 353.0[M+H]⁺, ESI pos.

### Step 6: 2-[3-(Difluoromethyl)-5-methyl-pyrazol-1-yl]-4-[6-[(6-methylpyridazin-3-yl) amino] pyrazolo [1,5-a]pyrimidin-3-yl]benzaldehyde

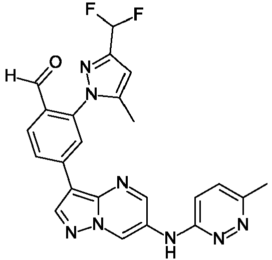

To a colorless solution of 2-[3-(difluoromethyl)-5-methyl-pyrazol-1-yl]-4-(4,4,5,5-tetramethyl-1,3,2-dioxaborolan-2-yl)benzaldehyde (308.56 mg, 0.85 mmol, 1.2 eqruiv.) in 1,4-dioxane (12 mL) were added 3-iodo-N-(6-methylpyridazin-3-yl)pyrazolo[1,5-a]pyrimidin-6-amine (6) 250.0 mg, 0.71 mmol, 1.0 equiv.), Pd(dppf)₂Cl₂•DCM (116 mg, 0.14 mmol, 0.2 equiv.), Na₂CO₃ (225.7 mg, 2.1 mmol, 3.0 equiv.) and water (2.5 mL). The brown suspension was stirred at 80 °C for 2 hours, then poured into water and extracted with ethyl acetate. The combined organics were washed with brine, dried over Na₂SO₄, filtered and concentrated. The crude product was purified by prep-HPLC using an 10 mmol NH₄Cl solution/acetonitrile gradient as eluent to provide the title compound (20.0 mg, 0.04 mmol, 6% yield) as yellow solid (30.0 mg, 0.07 mmol, 4% yield) as yellow solid. LC-MS: 461.2 [M+H]⁺, ESI pos.

### Step 7: 1-[2-[3-(Difluoromethyl)-5-methyl-pyrazol-1-yl]-4-[6-[(6-methylpyridazin-3-yl) amino] pyrazolo [1,5-a]pyrimidin-3-yl]phenyl]ethanol

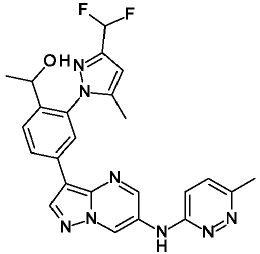

To a yellow solution of 2-[3-(difluoromethyl)-5-methyl-pyrazol-1-yl]-4-[6-[(6-methylpyridazin-3-yl)amino]pyrazolo[1,5-a]pyrimidin-3-yl]benzaldehyde (20.0 mg, 0.04 mmol, 1.0 equiv.) in THF (2 mL) was added methyl magnesium bromide (0.14 mL, 0.43 mmol, 10.0 equiv.) at -70 °C under a nitrogen atmosphere. The mixture was stirred at -70 °C for 2 hours, then quenched with saturated NH₄Cl solution and extracted with ethyl acetate. The combined organic layers were dried over Na₂SO₄, filtered and concentrated under reduced pressure to give the crude. The crude was purified by prep-HPLC using a 0.1% aqueous formic acid/acetonitrile gradient as eluent to give the title compound (3.59 mg, 0.01 mmol, 17.36% yield) as yellow solid after lyophilization. LC-MS: 459.2 [M+H-18]⁺, ESI pos.

¹H NMR (400 MHz, MeOH-d₄) δ = 9.97 (d, J = 2.3 Hz, 1H), 8.63 (d, J = 2.4 Hz, 1H), 8.50 (s, 1H), 8.26 (dd, J = 1.4, 8.3 Hz, 1H), 8.10 (d, J = 1.6 Hz, 1H), 7.81 (d, J = 8.2 Hz, 1H), 7.40 (d, J = 9.2 Hz, 1H), 7.15 (d, J = 9.0 Hz, 1H), 6.79 (t, J = 55.0 Hz, 1H), 6.55 (s, 1H), 4.53 - 4.42 (m, 1H), 2.55 (s, 3H), 2.28 (s, 3H), 1.35 (d, J = 6.5 Hz, 3H).

### Example 9 - Phosphorylation assay SIK1-3:

In the presence of SIK2 (resp. SIK1 or SIK3) and ATP the CHK-peptide (KKKVSRSGLYRSPSMPENLNRPR with C-terminal arginine amide modification) were phosphorylated at one of the four feasible serine's. Only one phosphorylation is observed under the assay conditions. 60 nl of each compound dilution series (12 point; dilution factor 3, generally 30 µM to 170 pM) in DMSO were transferred by acoustic dispensing to the assay plate and 30 minutes pre-incubated (ambient temperature) after the addition of 5 µl SIK1 (5 nM) resp. 5 µl SIK2 (0.5 nM) or 7 µl SIK3 (1.5 nM) in assay-buffer (12.5 mM HEPES (pH 7.0), 10 mM magnesium acetate, 0.005% BSA). 10 µM CHK-peptide solution and 5 µl of 100 µM ATP for SIK1 & SIK2 resp. 3 µl for SIK3 in assay-buffer were added and incubated ambient for 45 minutes. 40 µl of 0.125% formic acid in water were added to quench the reaction. RapidFire (RF) Mass Spectrometry was utilized for data generation as described below. The multiple charged species (3-5 charges) for the phosphorylated and non-phosphorylated form measured by MRM (Multiple Reaction Monitoring; API5000 or 6500+) or EIC (Extracted Ion Current; QToF) were summed up and the ratio calculated (sum phosphorylated species / sum all species) for data evaluation. Normalization was performed by Genedata software based on the non-inhibition control DMSO and the commercially available SIK inhibitor @ 1µM YKL-05-099 (CAS number 1936529-65-5). The results of the assay are expressed in half-maximal inhibitory concentrations (IC50s) and are summarized below in Table 1.

### RapidFire Setup:

Samples were aspirated by vacuum for max. 600ms and loaded to C4-cartridge (Agilent; #G9203A) for 3000ms@1.5ml/min with 0.1% formic acid in water. Afterwards samples were transferred to the API5000 (API6500+) or QToF mass spectrometer for 4000ms@1.25ml/min with 90% acetonitrile; 10% water; 0.007% TFA; 0.093 formic acid. The cartridge was reconditioned for additional 500ms with 0.1% formic acid in water.

### MS-Setup Sciex API5000/API6500+:

All MS analyses using the following MS-setup in MRM mode: Electrospray positive; Ion Spray Voltage: 4000V; Temperature: 550 °C; Collision Gas: 5; Curtain Gas: 15; Gas 1: 40; Gas 2: 42; EP: 10. DP = declustering potential; CE = collision energy; CXP = cell exit potential.

| Name | Q1 (m/z) | Q3 (m/z) | Time(ms) | DP (V) | CE (V) | CXP (V) |
|---|---|---|---|---|---|---|
| CHK(4+) | 676.0 | 84.2 | 50 | 46 | 99 | 18 |
| CHK(5+) | 541.0 | 84.2 | 50 | 51 | 71 | 36 |
| pCHK(4+) | 696.0 | 84.2 | 50 | 66 | 105 | 18 |
| pCHK(5+) | 557.0 | 84.2 | 50 | 51 | 53 | 4 |

### MS-Setup Agilent QToF 6545

All MS analyses using the following MS-setup in Mode MS: Dual AJS Electrospray positive; VCap: 3000V; Drying & Sheath gas: 340 °C@8l/min; Nebulizer: 60psig; Nozzle Voltage: 2000V; Fragmentor: 130V; Skimmer: 35V; Oct1 RF Vpp: 700V; Ref masses on@5spectra/s

**Table 1: IC50 values for inhibition of SIK1, SIK2 and SIK3:**

| Name | EIC (m/z) | Width |
|---|---|---|
| CHK(3+) | 900.8345 | 50ppm |
| CHK(4+) | 675.8789 | 50ppm |
| CHK(5+) | 540.9048 | 50ppm |
| pCHK(3+) | 927.4961 | 50ppm |
| pCHK(4+) | 695.8747 | 50ppm |

| **Example** | **SIK1 RF IC50 (µM)** | **SIK2 RF IC50 (µM)** | **SIK3 RF IC50 (µM)** |
|---|---|---|---|
| **1** | 2.800 | 0.016 | 0.002 |
| **2** | 0.616 | 0.001 | 0.0003 |
| **3** | 16.641 | 0.207 | 0.027 |
| **4** | 18.556 | 0.007 | 0.207 |
| **5** | 14.630 | 0.106 | 0.012 |
| **6** | ≥30.000 | 0.152 | 0.366 |
| **7** | 6.012 | 0.021 | 0.006 |
| **8** | ≥30.000 | 0.052 | 0.004 |

### Example A

Film coated tablets containing the following ingredients can be manufactured in a conventional manner:

| Ingredients | Per tablet | |
|---|---|---|
| **Kernel:** | | |
| Compound of formula (I) or a pharmaceutically acceptable salt thereof | 10.0 mg | 200.0 mg |
| Microcrystalline cellulose | 23.5 mg | 43.5 mg |
| Lactose hydrous | 60.0 mg | 70.0 mg |
| Povidone K30 | 12.5 mg | 15.0 mg |
| Sodium starch glycolate | 12.5 mg | 17.0 mg |
| Magnesium stearate | 1.5 mg | 4.5 mg |
| (Kernel Weight) | 120.0 mg | 350.0 mg |

| **Film Coat:** | | |
|---|---|---|
| Hydroxypropyl methyl cellulose | 3.5 mg | 7.0 mg |
| Polyethylene glycol 6000 | 0.8 mg | 1.6 mg |
| Talc | 1.3 mg | 2.6 mg |
| Iron oxide (yellow) | 0.8 mg | 1.6 mg |
| Titan dioxide | 0.8 mg | 1.6 mg |

The active ingredient is sieved and mixed with microcrystalline cellulose and the mixture is granulated with a solution of polyvinylpyrrolidone in water. The granulate is then mixed with sodium starch glycolate and magnesium stearate and compressed to yield kernels of 120 or 350 mg respectively. The kernels are lacquered with an aq. solution / suspension of the above mentioned film coat.

### Example B

Capsules containing the following ingredients can be manufactured in a conventional manner:

| Ingredients | Per capsule |
|---|---|
| Compound of formula (I) or a pharmaceutically acceptable salt thereof | 25.0 mg |
| Lactose | 150.0 mg |
| Maize starch | 20.0 mg |
| Talc | 5.0 mg |

The components are sieved and mixed and filled into capsules of size 2.

### Example C

Injection solutions can have the following composition:

| | |
|---|---|
| Compound of formula (I) or a pharmaceutically acceptable salt thereof | 3.0 mg |
| Polyethylene glycol 400 | 150.0 mg |
| Acetic acid | q.s. ad pH 5.0 |
| Water for injection solutions | ad 1.0 ml |

The active ingredient is dissolved in a mixture of Polyethylene glycol 400 and water for injection (part). The pH is adjusted to 5.0 by addition of acetic acid. The volume is adjusted to 1.0 ml by addition of the residual amount of water. The solution is filtered, filled into vials using an appropriate overage and sterilized.

## Claims

1. A compound of formula (I) 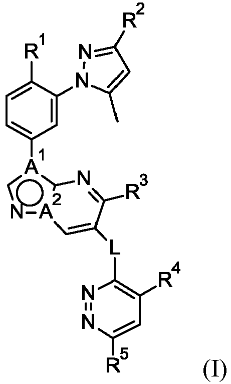 wherein
A¹ is -C- or -N-;
A² is -C- or -N-; with the proviso that if A¹ is -C-, then A² is -N-; and the proviso that if A¹ is - N-, then A² is -C-;
R¹ is cyano, hydroxyalkyl, alkylcarbonyl, haloalkoxy, hydroxy, alkoxy, halogen, haloalkyl, alkyl or cycloalkyl;
R² is cyano, hydroxyalkyl, haloalkoxy, hydroxy, alkoxy, halogen, haloalkyl or alkyl;
R³ is alkoxy, alkyl, haloalkyl, haloalkoxy, halogen or hydrogen;
R⁴ is hydrogen, alkyl, alkoxy, haloalkyl, alkylaminocarbonyl or dialkylaminocarbonyl;
R⁵ is hydrogen, alkyl, halogen, haloalkyl or alkoxy; and
L is -O- or -NH-;
or a pharmaceutically acceptable salt thereof.

2. The compound according to claim 1, wherein R¹ is cyano or hydroxyalkyl, in particular wherein R¹ is cyano or hydroxyethyl.

3. The compound according to claim 1 or 2, wherein R² is cyano, haloalkyl or haloalkoxy, in particular wherein R² is cyano, difluoromethyl or difluoromethoxy.

4. The compound according to any one of claims 1 to 3, wherein R³ is hydrogen or alkoxy, in particular wherein R³ is hydrogen or methoxy.

5. The compound according to any one of claims 1 to 4, wherein R⁴ is hydrogen or dialkylaminocarbonyl, in particular wherein R⁴ is hydrogen or dimethylaminocarbonyl.

6. The compound according to any one of claims 1 to 5, wherein R⁵ is hydrogen or alkyl, in particular wherein R⁵ is hydrogen or methyl.

7. The compound according to any one of claims 1 to 6, wherein A¹ is -C- and A² is -N-.

8. The compound according to any one of claims 1 to 7, wherein A¹ is -N- and A² is -C-.

9. The compound according to any one of claims 1 to 8, wherein L is -NH-.

10. The compound of formula (I) according to any one of claims 1 to 9, selected from
1-[2-(1-hydroxyethyl)-5-[6-[(6-methylpyridazin-3-yl)amino]imidazo[4,5-b]pyridin-3-yl]phenyl]-5-methyl-pyrazole-3-carbonitrile;
1-[2-(1-hydroxyethyl)-5-[6-[(6-methylpyridazin-3-yl)amino]pyrazolo[1,5-a]pyrimidin-3-yl]phenyl]-5-methyl-pyrazole-3-carbonitrile;
1-[2-[3-(difluoromethyl)-5-methyl-pyrazol-1-yl]-4-[6-(pyridazin-3-ylamino)imidazo[4,5-b]pyridin-3-yl]phenyl]ethanol;
3-[[3-[4-cyano-3-[3-(difluoromethoxy)-5-methyl-pyrazol-1-yl]phenyl]-5-methoxy-imidazo[4,5-b]pyridin-6-yl]amino]-N,N,6-trimethyl-pyridazine-4-carboxamide;
1-[2-(1-hydroxyethyl)-5-[6-(6-methylpyridazin-3-yl)oxypyrazolo[1,5-a]pyrimidin-3-yl]phenyl]-5-methylpyrazole-3-carbonitrile;
3-[[3-[4-cyano-3-[3-(difluoromethoxy)-5-methyl-pyrazol-1-yl]phenyl]imidazo[4,5-b]pyridin-6-yl]amino]-N,N,6-trimethyl-pyridazine-4-carboxamide;
1-[3-fluoro-2-(1-hydroxyethyl)-5-[6-[(6-methylpyridazin-3-yl)amino]imidazo[4,5-b]pyridin-3-yl]phenyl]-5-methyl-pyrazole-3-carbonitrile; and
1-[2-[3-(difluoromethyl)-5-methyl-pyrazol-1-yl]-4-[6-[(6-methylpyridazin-3-yl)amino]pyrazolo[1,5-a]pyrimidin-3-yl]phenyl]ethanol;
or a pharmaceutically acceptable salt thereof.

11. A process for the preparation of a compound according to any one of claims 1 to 10, comprising one of the following steps:
(a) the reaction of a compound of formula (B1) 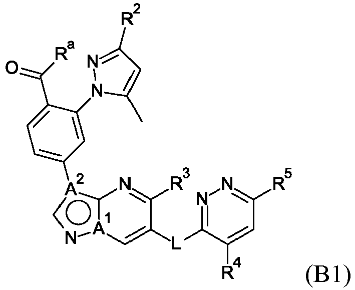 with a suitable reduction agent in presence of a suitable solvent,
(b) the reaction of a compound of formula (B2) 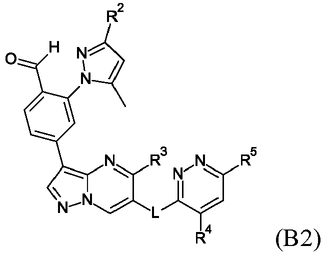 with a suitable Grignard reagent in presence of a suitable solvent,
(c) the reaction of a compound of formula (B3) 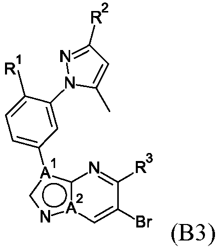
with a compound of formula (B4) 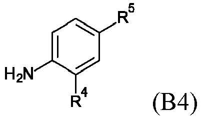
in presence of a suitable base, a suitable catalyst and a suitable solvent, or
(d) the reaction of a compound of formula (B5) or (B5) 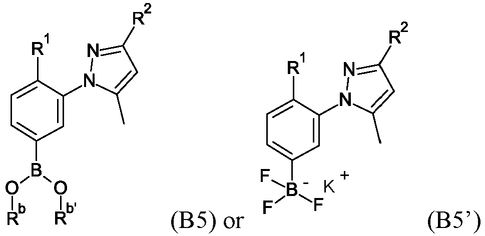
with a compound of formula (B6) 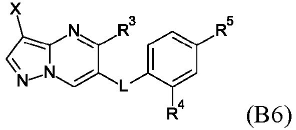
in presence of a suitable solvent,
wherein L, A¹, A², R¹, R², R³, R⁴ and R⁵ are as defined in any one of claims 1 to 9; X is halogen; R^{a} is alkyl, R^{b} and R^{b}' are independently selected from hydrogen and alkyl, or R^{b} and R^{b}' together with the oxygen atoms to which they are attached to form a five to six membered ring with the remaining ring members being carbon atoms optionally substituted with one or two alkyl substituents.

12. A compound of formula (I) according to any one of claims 1 to 10 or a pharmaceutically acceptable salt thereof, for use as therapeutically active substance.

13. A pharmaceutical composition comprising a compound of formula (I) according to any one of claims 1 to 10 or a pharmaceutically acceptable salt thereof, and a therapeutically inert carrier.

14. A compound of formula (I) according to any one of claims 1 to 10 or a pharmaceutically acceptable salt thereof, for use in the treatment or prophylaxis of rheumatoid arthritis, juvenile rheumatoid arthritis, non-alcoholic steatohepatitis (NASH), primary sclerosing cholangitis, giant cell vasculitis, inflammatory bowel diseases (IBD), atherosclerosis, type 2 diabetes or glomerulonephritis.

## Patentansprüche

1. Verbindung der Formel (I) 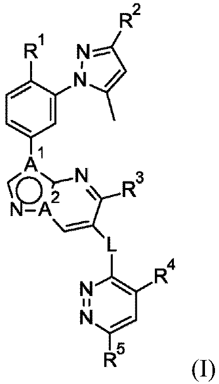 wobei
A¹ -C- oder -N- ist;
A² -C- oder -N- ist; mit der Maßgabe, dass, wenn A¹ -C- ist, dann A² -N- ist; und der Maßgabe, dass, wenn A¹ -N- ist, dann A² -C- ist;
R¹ Cyano, Hydroxyalkyl, Alkylcarbonyl, Halogenalkoxy, Hydroxy, Alkoxy, Halogen, Halogenalkyl, Alkyl oder Cycloalkyl ist;
R² Cyano, Hydroxyalkyl, Halogenalkoxy, Hydroxy, Alkoxy, Halogen, Halogenalkyl oder Alkyl ist;
R³ Alkoxy, Alkyl, Halogenalkyl, Halogenalkoxy, Halogen oder Wasserstoff ist;
R⁴ Wasserstoff, Alkyl, Alkoxy, Halogenalkyl, Alkylaminocarbonyl oder Dialkylaminocarbonyl ist;
R⁵ Wasserstoff, Alkyl, Halogen, Halogenalkyl oder Alkoxy ist; und
L -O- oder -NH- ist;
oder ein pharmazeutisch verträgliches Salz davon.

2. Verbindung nach Anspruch 1, wobei R¹ Cyano oder Hydroxyalkyl ist, insbesondere wobei R¹ Cyano oder Hydroxyethyl ist.

3. Verbindung nach Anspruch 1 oder 2, wobei R² Cyano, Halogenalkyl oder Halogenalkoxy ist, insbesondere wobei R² Cyano, Difluormethyl oder Difluormethoxy ist.

4. Verbindung nach einem der Ansprüche 1 bis 3, wobei R³ Wasserstoff oder Alkoxy ist, insbesondere wobei R³ Wasserstoff oder Methoxy ist.

5. Verbindung nach einem der Ansprüche 1 bis 4, wobei R⁴ Wasserstoff oder Dialkylaminocarbonyl ist, insbesondere wobei R⁴ Wasserstoff oder Dimethylaminocarbonyl ist.

6. Verbindung nach einem der Ansprüche 1 bis 5, wobei R⁵ Wasserstoff oder Alkyl ist, insbesondere wobei R⁵ Wasserstoff oder Methyl ist.

7. Verbindung nach einem der Ansprüche 1 bis 6, wobei A¹ -C- ist und A² -N- ist.

8. Verbindung nach einem der Ansprüche 1 bis 7, wobei A¹ -N- ist und A² -C- ist.

9. Verbindung nach einem der Ansprüche 1 bis 8, wobei L -NH- ist.

10. Verbindung der Formel (I) nach einem der Ansprüche 1 bis 9, ausgewählt aus
1-[2-(1-Hydroxyethyl)-5-[6-[(6-methylpyridazin-3-yl)amino]imidazo[4,5-b]pyridin-3-yl]phenyl]-5-methylpyrazol-3-carbonitril;
1-[2-(1-Hydroxyethyl)-5-[6-[(6-methylpyridazin-3-yl)amino]pyrazolo[1,5-a]pyrimidin-3-yl]phenyl]-5-methylpyrazol-3-carbonitril;
1-[2-[3-(Difluormethyl)-5-methylpyrazol-1-yl]-4-[6-(pyridazin-3-ylamino)imidazo[4,5-b]pyridin-3-yl]phenyl]ethanol;
3-[[3-[4-Cyano-3-[3-(difluormethoxy)-5-methylpyrazol-1-yl]phenyl]-5-methoxyimidazo[4,5-b]pyridin-6-yl]amino]-N,N,6-trimethylpyridazin-4-carboxamid;
1-[2-(1-Hydroxyethyl)-5-[6-(6-methylpyridazin-3-yl)oxypyrazolo[1,5-a]pyrimidin-3-yl]phenyl]-5-methylpyrazol-3-carbonitril;
3-[[3-[4-Cyano-3-[3-(difluormethoxy)-5-methylpyrazol-1-yl]phenyl]imidazo[4,5-b]pyridin-6-yl]amino]-N,N,6-trimethylpyridazin-4-carboxamid;
1-[3-Fluor-2-(1-hydroxyethyl)-5-[6-[(6-methylpyridazin-3-yl)amino]imidazo[4,5-b]pyridin-3-yl]phenyl]-5-methylpyrazol-3-carbonitril; und
1-[2-[3-(Difluormethyl)-5-methylpyrazol-1-yl]-4-[6-[(6-methylpyridazin-3-yl)amino]pyrazolo[1,5-a]pyrimidin-3-yl]phenyl]ethanol;
oder ein pharmazeutisch verträgliches Salz davon.

11. Verfahren zur Herstellung einer Verbindung nach einem der Ansprüche 1 bis 10, umfassend einen der folgenden Schritte:
(a) die Reaktion einer Verbindung der Formel (B1) 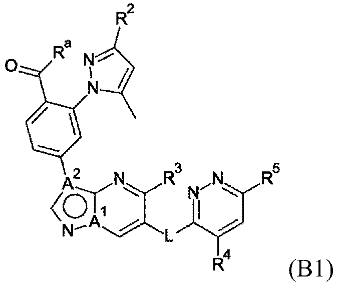 mit einem geeigneten Reduktionsmittel in Gegenwart eines geeigneten Lösungsmittels,
(b) die Reaktion einer Verbindung der Formel (B2) 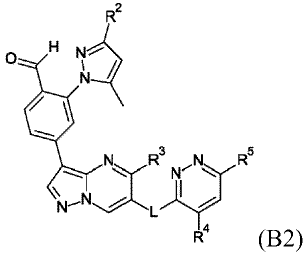 mit einem geeigneten Grignard-Reagens in Gegenwart eines geeigneten Lösungsmittels,
(c) die Reaktion einer Verbindung der Formel (B3) 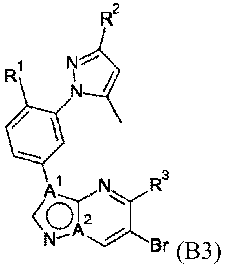
mit einer Verbindung der Formel (B4) 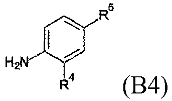
in Gegenwart einer geeigneten Base, eines geeigneten Katalysators und eines geeigneten Lösungsmittels, oder
(d) die Reaktion einer Verbindung der Formel (B5) oder (B5') 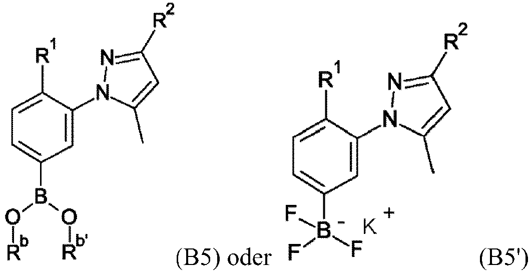
mit einer Verbindung der Formel (B6) 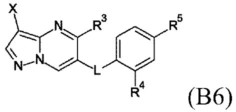
in Gegenwart eines geeigneten Lösungsmittels,
wobei L, A¹, A², R¹, R², R³, R⁴ und R⁵ wie in einem der Ansprüche 1 bis 9 definiert sind, X Halogen ist; R^{a} Alkyl ist, R^{b} und R^{b'} unabhängig voneinander aus Wasserstoff und Alkyl ausgewählt sind oder R^{b} und R^{b'} zusammen mit den Sauerstoffatomen, an die sie gebunden sind, einen fünf- bis sechsgliedrigen Ring bilden, wobei die verbleibenden Ringglieder Kohlenstoffatome sind, die gegebenenfalls mit einem oder zwei Alkylsubstituenten substituiert sind.

12. Verbindung der Formel (I) nach einem der Ansprüche 1 bis 10 oder ein pharmazeutisch verträgliches Salz davon zur Verwendung als therapeutisch wirksame Substanz.

13. Pharmazeutische Zusammensetzung, umfassend eine Verbindung der Formel (I) nach einem der Ansprüche 1 bis 10 oder ein pharmazeutisch verträgliches Salz davon und einen therapeutisch inerten Träger.

14. Verbindung der Formel (I) nach einem der Ansprüche 1 bis 10 oder ein pharmazeutisch verträgliches Salz davon zur Verwendung bei der Behandlung oder Prophylaxe von rheumatoider Arthritis, juveniler rheumatoider Arthritis, nichtalkoholischer Steatohepatitis (NASH), primärer sklerosierender Cholangitis, Riesenzellvaskulitis, entzündlichen Darmerkrankungen (IBD), Atherosklerose, Typ-2-Diabetes oder Glomerulonephritis.

## Revendications

1. Composé de formule (I) 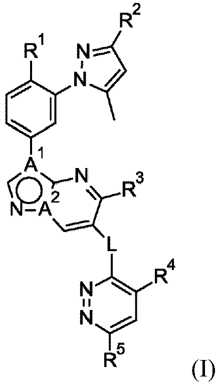 dans lequel
A¹ représente -C- ou -N- ;
A² représente -C- ou -N- ; à condition que si A¹ représente -C-, alors A² représente -N- ; et à condition que si A¹ représente -N-, alors A² représente -C- ;
R¹ représente un cyano, un hydroxyalkyle, un alkylcarbonyle, un halogénoalcoxy, un hydroxy, un alcoxy, un halogène, un halogénoalkyle, un alkyle ou un cycloalkyle ;
R² représente un cyano, un hydroxyalkyle, un halogénoalcoxy, un hydroxy, un alcoxy, un halogène, un halogénoalkyle ou un alkyle ;
R³ représente un alcoxy, un alkyle, un halogénoalkyle, un halogénoalcoxy, un halogène ou un hydrogène ;
R⁴ représente un hydrogène, un alkyle, un alcoxy, un halogénoalkyle, un alkylaminocarbonyle ou un dialkylaminocarbonyle ;
R⁵ représente un hydrogène, un alkyle, un halogène, un halogénoalkyle ou un alcoxy ; et
L représente -O- ou -NH- ;
ou un sel pharmaceutiquement acceptable de celui-ci.

2. Composé selon la revendication 1, dans lequel R¹ représente un cyano ou un hydroxyalkyle, en particulier dans lequel R¹ représente un cyano ou un hydroxyéthyle.

3. Composé selon la revendication 1 ou 2, dans lequel R² représente un cyano, un halogénoalkyle ou un halogénoalcoxy, en particulier dans lequel R² représente un cyano, un difluorométhyle ou un difluorométhoxy.

4. Composé selon l'une quelconque des revendications 1 à 3, dans lequel R³ représente un hydrogène ou un alcoxy, en particulier dans lequel R³ représente un hydrogène ou un méthoxy.

5. Composé selon l'une quelconque des revendications 1 à 4, dans lequel R⁴ représente un hydrogène ou un dialkylaminocarbonyle, en particulier dans lequel R⁴ représente un hydrogène ou un diméthylaminocarbonyle.

6. Composé selon l'une quelconque des revendications 1 à 5, dans lequel R⁵ représente un hydrogène ou un alkyle, en particulier dans lequel R⁵ représente un hydrogène ou un méthyle.

7. Composé selon l'une quelconque des revendications 1 à 6, dans lequel A¹ représente -C- et A² représente -N-.

8. Composé selon l'une quelconque des revendications 1 à 7, dans lequel A¹ représente -N- et A² représente -C-.

9. Composé selon l'une quelconque des revendications 1 à 8, dans lequel L représente -NH-.

10. Composé de formule (I) selon l'une quelconque des revendications 1 à 9, choisi parmi
le 1-[2-(1-hydroxyéthyl)-5-[6-[(6-méthylpyridazin-3-yl)amino]imidazo[4,5-b]pyridin-3-yl]phényl]-5-méthyl-pyrazole-3-carbonitrile ;
le 1-[2-(1-hydroxyéthyl)-5-[6-[(6-méthylpyridazin-3-yl)amino]pyrazolo[1,5-a]pyrimidin-3-yl]phényl]-5-méthyl-pyrazole-3-carbonitrile ;
le 1-[2-[3-(difluorométhyl)-5-méthyl-pyrazol-1-yl]-4-[6-(pyridazin-3-ylamino)imidazo[4,5-b]pyridin-3-yl]phényl]éthanol ;
le 3-[[3-[4-cyano-3-[3-(difluorométhoxy)-5-méthyl-pyrazol-1-yl]phényl]-5-méthoxy-imidazo[4,5-b]pyridin-6-yl]amino]-N,N,6-triméthyl-pyridazine-4-carboxamide ;
le 1-[2-(1-hydroxyéthyl)-5-[6-(6-méthylpyridazin-3-yl)oxypyrazolo[1,5-a]pyrimidin-3-yl]phényl]-5-méthylpyrazole-3-carbonitrile ;
le 3-[[3-[4-cyano-3-[3-(difluorométhoxy)-5-méthyl-pyrazol-1-yl]phényl]imidazo[4,5-b]pyridin-6-yl]amino]-N,N,6-triméthyl-pyridazine-4-carboxamide ;
le 1-[3-fluoro-2-(1-hydroxyéthyl)-5-[6-[(6-méthylpyridazin-3-yl)amino]imidazo[4,5-b]pyridin-3-yl]phényl]-5-méthyl-pyrazole-3-carbonitrile ; et
le 1-[2-[3-(difluorométhyl)-5-méthyl-pyrazol-1-yl]-4-[6-[(6-méthylpyridazin-3-yl)amino]pyrazolo[1,5-a]pyrimidin-3-yl]phényl]éthanol ;
ou un sel pharmaceutiquement acceptable de ceux-ci.

11. Procédé pour la préparation d'un composé selon l'une quelconque des revendications 1 à 10, comprenant l'une des étapes suivantes :
(a) la réaction d'un composé de formule (B1) 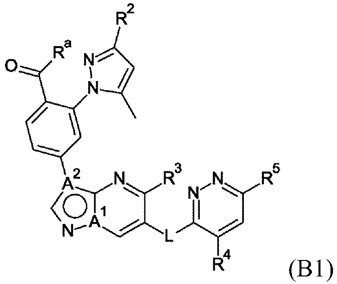 avec un agent de réduction approprié en présence d'un solvant approprié,
(b) la réaction d'un composé de formule (B2) 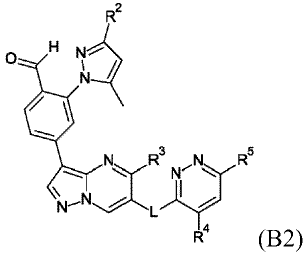 avec un réactif de Grignard approprié en présence d'un solvant approprié,
(c) la réaction d'un composé de formule (B3) 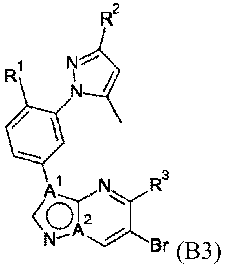
avec un composé de formule (B4) 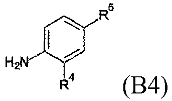
en présence d'une base appropriée, d'un catalyseur approprié et d'un solvant approprié, ou
(d) la réaction d'un composé de formule (B5) ou (B5') 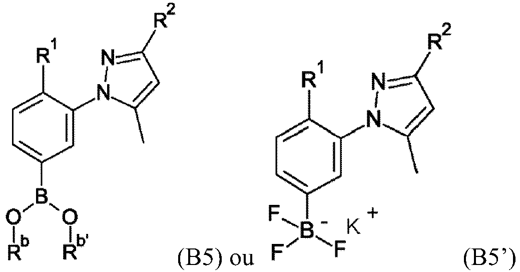
avec un composé de formule (B6) 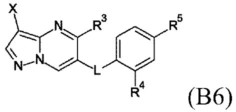
en présence d'un solvant approprié,
dans lequel L, A¹, A², R¹, R², R³, R⁴ et R⁵ sont tels que définis dans l'une quelconque des revendications 1 à 9 ; X représente un halogène ; R^{a} représente un alkyle, R^{b} et R^{b'} sont indépendamment choisis parmi un hydrogène et un alkyle, ou R^{b} et R^{b'} conjointement avec les atomes d'oxygène auxquels ils sont liés forment un cycle à cinq à six chaînons, les chaînons du cycle restants étant des atomes de carbone éventuellement substitués par un ou deux substituants alkyle.

12. Composé de formule (I) selon l'une quelconque des revendications 1 à 10 ou un sel pharmaceutiquement acceptable de celui-ci, pour une utilisation comme substance thérapeutiquement active.

13. Composition pharmaceutique comprenant un composé de formule (I) selon l'une quelconque des revendications 1 à 10 ou un sel pharmaceutiquement acceptable de celui-ci, et un véhicule thérapeutiquement inerte.

14. Composé de formule (I) selon l'une quelconque des revendications 1 à 10 ou un sel pharmaceutiquement acceptable de celui-ci, pour une utilisation dans le traitement ou la prophylaxie de la polyarthrite rhumatoïde, de la polyarthrite rhumatoïde juvénile, de la stéatohépatite non alcoolique (SHNA), de la cholangite sclérosante primitive, de la vascularite à cellules géantes, des maladies inflammatoires de l'intestin (MII), de l'athérosclérose, du diabète de type 2 ou de la glomérulonéphrite.
